(19) Europäisches Patentamt / European Patent Office / Office européen des brevets

(11) EP 3 606 517 B1

(12) # EUROPEAN PATENT SPECIFICATION

(45) Date of publication and mention of the grant of the patent:
**25.05.2022 Bulletin 2022/21**

(21) Application number: **17715946.4**

(22) Date of filing: **07.04.2017**

(51) International Patent Classification (IPC):
***A61K 31/366*** (2006.01) ***A61P 11/00*** (2006.01)
***A61P 11/06*** (2006.01) ***A61K 9/00*** (2006.01)
***A61K 9/06*** (2006.01) ***A61K 9/20*** (2006.01)
***A61K 9/48*** (2006.01)

(52) Cooperative Patent Classification (CPC):
**A61K 31/366; A61K 9/0014; A61K 9/0019; A61K 9/0075; A61K 9/0078; A61K 9/06; A61K 9/2018; A61K 9/4866; A61P 11/00; A61P 11/06**

(86) International application number:
**PCT/EP2017/058349**

(87) International publication number:
**WO 2018/184687 (11.10.2018 Gazette 2018/41)**

(54) **COUMARIN DERIVATIVE AS ANTI-ASTHMATIC AGENT, PHARMACEUTICAL COMPOSITION THEREOF AND USE**

CUMARINDERIVAT ALS ANTI-ASTHMATISCHER WIRKSTOFF, PHARMAZEUTISCHE ZUSAMMENSETZUNG DARAUS UND VERWENDUNG

DÉRIVÉ DE COUMARINE UTILE EN TANT QU'AGENT ANTI-ASTHMATIQUE, COMPOSITION PHARMACEUTIQUE LE CONTENANT ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(43) Date of publication of application:
**12.02.2020 Bulletin 2020/07**

(73) Proprietor: **I-NOVA MEDICINSKA ISTRAZIVANJA d.o.o.**
**32000 Vukovar (HR)**

(72) Inventors:
- **TRKOVNIK, Mladen**
  **10000 Zagreb (HR)**
- **CACIC, Milan**
  **32000 Vukovar (HR)**
- **RIZVANI, Jonuz**
  **10000 Zagreb (HR)**

(74) Representative: **Bihar, Zeljko**
**Admoveo d.o.o.**
**Gracanska cesta 111**
**10000 Zagreb (HR)**

(56) References cited:
**WO-A1-2005/010006** **WO-A1-2005/095411**
**WO-A1-2016/156888**

- **XIONG Y Y ET AL: "Attenuation of airway hyperreactivity and T helper cell type 2 responses by coumarins from Peucedanum praeruptorum Dunn in a murine model of allergic airway inflammation", JOURNAL OF ETHNOPHARMACOLOGY, ELSEVIER IRELAND LTD, IE, 1 May 2013 (2013-05-01), pages 314-321, XP018507944, ISSN: 0378-8741, DOI: 10.1016/J.JEP.2012.02.037**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 606 517 B1



(Cont. next page)

- GRIMM E L ET AL: "Substituted coumarins as potent 5-lipoxygenase inhibitors", BIOORGANIC & MEDICINAL CHEMISTRY LETTERS, PERGAMON, AMSTERDAM, NL, vol. 16, no. 9, 1 May 2006 (2006-05-01), pages 2528-2531, XP027966269, ISSN: 0960-894X [retrieved on 2006-05-01]

## Description

### Technical Field

The invention relates to novel pharmacological uses of pharmaceutical composition based on coumarin derivative 7,9-dihydroxy-3-(4,5,7-trihydroxy-2-oxo-2*H*-chromene-3-yl)-4*H*-furo[3,2-c]chromen-4-one (**1**) as an active pharmaceutical ingredient (API).

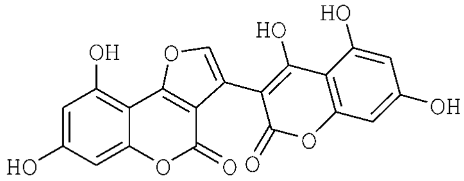

**1**

### Technical Problem

Technical problem is related to safe and efficient therapy for treatment of asthma and other inflammatory respiratory diseases.
The technical problem that is solved by the present invention is based on the compound **1** and its use as active pharmaceutical ingredient (API), in the form of various pharmaceutical final dosage forms suitable for therapeutic use.

### Previous State of Art

Asthma or bronchial asthma is a widespread chronic disease of the air passages of the lungs which inflames and narrows them. It is characterized by recurrent attacks of breathlessness, wheezing, bronchospasm, coughing, and chest tightness. Episodes of asthma attacks can vary in severity and frequency from person to person. These symptoms may occur several times during a day or week in affected individuals, and for some people become worse during physical activity or at night. World Health Organization (WHO) estimates that 235 million people worldwide currently suffer from this disease. The fundamental causes of asthma are not fully understood. Asthma cannot be cured, but adequate management can control the disease and enable people to enjoy a good quality of life; see literature reference 1:

1) WHO: Asthma, Fact Sheet, World Health Organisation (2013).

The therapy of asthma includes short-term medications which are used to relieve symptoms. An example of such drugs is salbutamol or terbutaline. The progression of severe asthma can be controlled by long-term administration of anti-inflammatory steroids like beclomethasone dipropionate and fluticasone propionate or furoate, etc; see literature reference 2:
2) M. Salter, K. Biggadike, J. L. Matthews, M. R. West, M. V. Haase, S. N. Farrow, I. J. Uings, D. W. Gray: Pharmacological properties of the enhanced-affinity glucocorticoid fluticasone furoate in vitro and in vivo model of respiratory inflammatory disease, Am. J. Physiol. Lung Cell Mol. Physiol. 293 (2007) L660-L667.
Whilst some anti-asthmatic drugs are applied by inhalation, another one are administered orally or topically, e.g. prednisone or prednisolone, theophylline, as well as leukotriene antagonists and inhibitors like montelukast, zafirlukast, etc; see literature reference 3:
3) K. F. Kerrebijn: Use of topical corticosteroids in the treatment of childhood asthma, Am. Rev. Respir. Dis. 141 (1990) S77-S81.
Beside asthma, examples of other inflammatory respiratory diseases are: allergic rhinitis and sinusitis, acute respiratory disease syndrome (ARDS), and chronic obstructive pulmonary disease (COPD); see literature reference 4:
4) WHO: Chronic obstructive pulmonary disease (COPD), Fact Sheet, World Health Organisation (2016).
The therapy of these diseases is, beside antihistamines and nasal decongestants for allergic rhinitis and sinusitis are also based on above mentioned anti-inflammatory drugs.
Coumarins are a well-known class of chemical substances of various valuable pharmacological activities; for instance see literature reference 5:
5) F. Borges, F. Roleira, N. Milhazes, L. Santana, E. Uriarte: Simple Coumarins and Analogues in Medicinal Chemistry: Occurence, Synthesis and Biological Activity, Curr. Med. Chem. 12 (2005) 887-916.
4-Hydroxycoumarins undergo an aldol-type condensation reaction with aldehydes yielding corresponding 1-hydroxyalkyl derivatives of parent aldehyde at 3-position of starting 4-hydroxycoumarin. This type of condensation products was already studied from both synthetic and pharmacological points of view. Thus, Merčep and coworkers disclosed the condensation products of various 4-hydroxy coumarins with glyoxal yielding several compounds of general formula **I** of anti-inflammatory activity. Furthermore, in another application they described 2,7,9-trihydroxy-3-(4,5,7-trihydroxy-2-oxo-2*H*-chromene-3-yl)-2,3-dihydro-4*H*-furo[3,2-*c*]chromen-4-one (**2**), for which they also reported anti-inflammatory activity; see literature references 6 and 7:

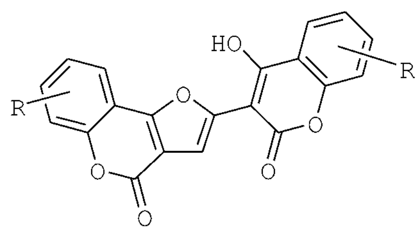

I

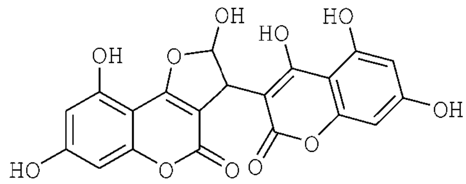

2

6) M. Merčep, M. Mesić, B. Hrvačić, I. J. Elenkov, I. Malnar, S. Marković, L. Šimičić, A. Cempuh Klonkay: Substituted furochromenes, preparation thereof and their anti-inflammatory action, WO2005/010007A1, Pliva - Istraživački institut d.o.o.;

7) M. Merčep, M. Mesic, B. Hrvačić, I. J. Elenkov, I. Malnar: Furochromene derivative with anti-inflammatory activity, WO2005/095411A1, Pliva - Istraživacki institut d.o.o.

Additionally, Ivezic disclosed alkoxy-derivatives of corresponding bis-condensation products of general formula **II**.

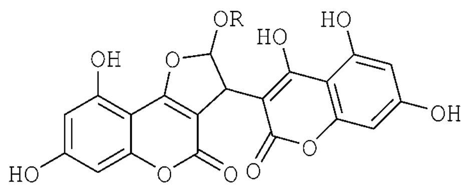

R= $CH_3$, $C_2H_5$

II

The latter compounds were tested on antiviral activity including the anti-HIV activity; see literature reference 8: 8) Z. Ivezic: [Synthesis of novel hydroxycoumarin derivatives as possible HIV-1 protease inhibitors (in Croatian)] Ph.D. Thesis (2000) Pliva Inc., Zagreb, Croatia; Faculty of Science, University of Zagreb, Croatia.

These are relatively the most similar compounds from the prior art, but compound **2** and compounds of general formula **II** can establish more interactions with various enzyme active sites, e.g. at least one hydrogen-bond more than the compound **1** (from the present invention) in the same situation; **2** as hydrogen-bond donor thanks to the hydrogen atom at 2-hydroxy-furane moiety, and **II** as hydrogen-bond acceptor due to proton-accepting potential of oxygen atom of 2-alkoxy-furane moiety.

Thus, from standpoint of pharmaceutical chemistry and pharmacology, there exist significant structural differences between the known compounds from the classes **I** and **II** or compound **2** versus compound **1** from the present invention.

According to our best knowledge, the closest prior art is our previous patent application wherein we disclosed the coumarin derivative 7,9-dihydroxy-3-(4,5,7-trihydroxy-2-oxo-2*H*-chromene-3-yl)-4*H*-furo[3,2-*c*]chromen-4-one (**1**), its synthesis, various pharmaceutical final dosage forms, and pharmacological activity as therapeutic agent for treatment of either viral or, specifically, immunodeficiency virus type-1 (HIV-1) infections; see literature reference 9:
9) M. Trkovnik, M. Čačić, J. Rizvani: Coumarin derivative as antiviral agent, pharmaceutical composition thereof, its preparation and use, WO2016/156888A1; Applicant: I-NOVA Medicinska istraživanja d.o.o.

Additionally, the use of compound **1** through its various pharmaceutical final dosage forms for therapy of asthma and other inflammatory respiratory diseases, to our best knowledge, has not been disclosed in the prior art.

## Summary of the Invention

The invention discloses novel pharmacological uses of pharmaceutical composition based on the coumarin derivative 7,9-dihydroxy-3-(4,5,7-trihydroxy-2-oxo-2*H*-chromene-3-yl)-4*H*-furo[3,2-*c*]chromen-4-one (**1**) as the active pharmaceutical ingredient (API); for use in the therapy of asthma or other inflammatory respiratory diseases such as allergic rhinitis and sinusitis, acute respiratory disease syndrome, and chronic obstructive pulmonary disease.

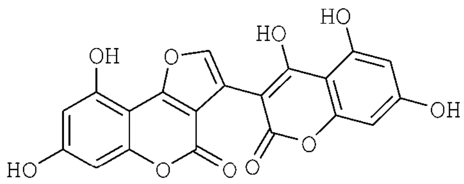

**1**

Also disclosed herein is the process for production of said pharmaceutical composition.

## Brief Description of the Drawing

**Figure 1** - Anti-inflammatory activity of 7,9-dihydroxy-3-(4,5,7-trihydroxy-2-oxo-2*H*-chromene-3-yl)-4*H*-furo[3,2-*c*]chromen-4-one (**1**) in the experimental *in vivo* model of croton oil-induced ear edema in mice:

(1) I bar represents the increasing of the weight of ear sample due to croton oil-induced ear edema (the negative control).
(2) II bar represents the increasing the weight of ear sample due to croton oil-induced ear edema in the presence of dexamethasone (DEXA), a model anti-inflammatory agent (the positive control).
(3) III bar represents the increasing of the weight of the ear sample due to croton oil-induced ear edema in the presence of the compound **1** from the present invention; and
(4) IV bar represents the change of the weight of untreated ear sample (the control).

**Figure 2** - Development of model of lung eosinophilia in mice and a treatment schedule.

**Figures 3A, 3B** - Differential cell count of bronchoalveolar lavage (BAL) cells from mice with allergic pulmonary inflammation challenged by ovalbumin (OVA). Bars show the number of total BAL cells and the various types of infiltrating inflammatory cells in the BAL of experimental mice 2 days after intranasal (i.n.) challenge with OVA. BAL cells were fixed to the slides by cytospin. A blinded differential cell count was performed and the absolute number of alveolar macrophages, eosinophils, lymphocytes, and neutrophils equals the total number of BAL cells per lung as percentage of that cell type. Mean ± SD values were calculated for each group; p values were calculated using the two-way ANOVA test. (*$p$<0.05; **$p$<0.01) in comparison to phosphate-buffered saline (PBS)-treated controls. (*$p$<0.05), in comparison to 0.1 M PBS-injected controls.

**Figure 4** - Lung histology of the OVA-challenged Balb/c mice. Histological examination of paraffin-embedded lung sections from PBS-treated OVA-challenged mice; beclomethasone treated OVA-challenged mice and compound **1** treated (preventively and therapeutically) OVA-challenged mice. Standard haematoxylin/eosin staining technique was used; see Example 3. Representative images are shown. Data are representative of at least 5 lungs. Original magnification 250x.

**Figure 5** - Quantitative measurement of airway mucus production in mice with allergic pulmonary inflammation that were treated with compound **1**. Examples of PAS staining on lung sections from PBS-treated OVA-challenged mice; beclomethasone treated OVA-challenged mice and compound **1** treated (preventively and therapeutically) OVA-challenged mice. Mucus-producing goblet cells (bright purple) are stained. Data are representative of at least 5 lungs. Original magnification 250×.

**Figure 6** - Serial sections of ovalbumin (OVA)-sensitised animals stained for IL-5 production in mice with allergic pulmonary inflammation that were treated with compound **1.** Groups represent examples of IL-5 staining on lung

sections from PBS-treated OVA-challenged mice; beclomethasone treated OVA-challenged mice and compound **1** treated (preventively and therapeutically) OVA-challenged mice. IL-5-producing cells (brown) are stained. The black arrows indicate the cells with IL-5 production. Data are representative of at least 5 lungs. Original magnification 400x.

**Figure 7** - Quantitative measurement of CCL11 production in mice with allergic pulmonary inflammation. Groups represent examples of CCL11 staining on lung sections from PBS-treated OVA-challenged mice; beclomethasone treated OVA-challenged mice and compound **1** treated (preventively and therapeutically) OVA-challenged mice. CCL11-producing cells (dark brown) are stained. The black arrows indicate the cells producing CCL11. Data are representative of at least 5 lungs. Original magnification 400x.

## Detailed Description

The present invention discloses novel pharmacological uses of pharmaceutical composition comprising:

(1) 7,9-dihydroxy-3-(4,5,7-trihydroxy-2-oxo-2*H*-chromene-3-yl)-4*H*-furo[3,2-c]chromen-4-one (**1**) or a pharmaceutically acceptable salt or hydrate thereof as active pharmaceutical ingredient;

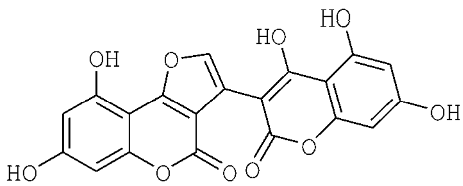

**1**

and
(2) one or more pharmaceutical excipients, required to yield final dosage forms suitable for therapeutic administration; for use in the therapy of asthma or other inflammatory respiratory diseases.

Other respiratory diseases are selected from the group consisting of:

(i) allergic rhinitis and sinusitis;
(ii) acute respiratory disease syndrome (ARDS); and
(iii) chronic obstructive pulmonary disease (COPD).

Also disclosed herein is the process for manufacturing of this pharmaceutical composition. The composition of the present invention can be in various final dosage forms that are selected by the manner of use and include the following forms:

(1) inhalation: spray solution, spray suspension, or inhalation powder;
(2) oral: tablets, capsules, granules, powder, divided powder, effervescent tablets, oral solution, oral suspension, syrup, lozenges, chewing gum;
(3) topical: ointment, cream, gel, liniment, lotion, poultice, therapeutic patch;
(4) parenteral: injection solution; as well as
(5) all other commonly used pharmaceutical final dosage forms, since compound **1** can be readily formulated in many different dosage forms.

The pharmaceutical excipient required to prepare final dosage forms is one or more substances selected from the group comprising:

(1) fillers; for solid dosage forms like tablets, capsules, powders, etc.;
(2) diluents; for liquid dosage forms such as syrup, liniment, lotion, etc.;
(3) emollients; for topical dosage forms like creams, ointments, etc.;
(4) emulsifiers;
(5) binders;
(6) disintegrants;

(7) lubricants;
(8) humectants;
(9) thickeners;
(10) chelating agents;
(11) preservatives;
(12) antioxidants; as well as
(13) all other classes of excipients that are commonly used in the pharmaceutical technology.

Filler is selected from the group comprising: lactose, saccharose, mannitol, sorbitol, maltitol, xylitol, dextrin, maltodextrin, starch, microcrystalline cellulose, inulin, calcium carbonate, mixtures of these substances, or other pharmaceutically acceptable fillers.

Diluent is selected from the group comprising: purified water; ethanol; 1,2-propylene glycol; glycerol; polyethyleneglycols (PEG) like PEG 400; plant oils like sunflower oil, sesame oil, or medium-chain triglycerides; mutually miscible or emulsifiable mixtures of these substances, or other pharmaceutically acceptable diluents.

Purified water that is used as the diluents in the composition of the present invention meets the requirements of European pharmacopoeia 8.0, p.3561-3562 for pharmaceutical water.

Emollient is selected from the group comprising: petroleum jelly; mineral oil; plant oils like almond, sunflower, or sesame oil; medium-chain triglycerides; natural or synthetic esters of monovalent alcohols with higher fatty acids like isopropyl myristate, jojoba oil, or beeswax; silicone oil; higher fatty acids like stearic acid; higher fatty alcohols like cetyl alcohol; mixtures of these substances, or other pharmaceutically acceptable emollients.

Emulsifier is selected from the group comprising: lanolin; ethoxylated lanolin; lanolin alcohols; ethoxylated lanolin alcohols; lecithin; hydrogenated lecithin; mono- and diesters of glycerol with higher fatty acids like glyceryl monostearate; sorbitan esters with higher fatty acids such as sorbitan monostearate; ethoxylated higher fatty alcohols or acids like polyoxyethylene(20) laurylether or polyoxyethylene(2) oleate, wherein numbers 20 and 2 represents the number of ethyleneglycol units; esters of ethoxylated sorbitan like polysorbate 60; water soluble soaps like sodium stearate; mixtures of these substances, or other pharmaceutically acceptable emulsifiers.

Binder is selected from the group comprising: glucose syrup; glucose-fructose syrup; honey; saccharose; lactose; gelatine; sorbitol; maltitol; xylitol; cellulose gums like hydroxypropyl methylcellulose (HPMC), hydroxypropyl cellulose (HPC), hydroxyethyl cellulose (HEC), methyl cellulose (MC), sodium carboxymethyl cellulose (NaCMC); synthetic polymers such as polyvinyl alcohol (PVA), polyacrylic acid (PAA) and its copolymers, polyvinylpyrrolidone (PVP); hyaluronic acid; various gums like gum arabic, xanthan gum, guar gum, tragacanth; alginic acid and its salts like sodium alginate; mixtures of these substances, or other pharmaceutically acceptable binders.

Disintegrant is selected from the group comprising: crosslinked polyvinylpyrrolidone (PVP); sodium starch glycolate; crosslinked sodium carboxymethylcellulose (NaCMC); modified starches; mixtures of these substances, or other pharmaceutically acceptable disintegrants.

Lubricant is selected from the group comprising: magnesium, calcium, aluminium, and zinc soaps, e.g. magnesium stearate; higher fatty acids like stearic acid; talc; colloidal silica (silicon dioxide); mixtures of these substances, or other pharmaceutically acceptable lubricants.

Humectant is selected from the group comprising: glycerol; 1,2-propylene glycol; hexylene glycol; liquid sorbitol; d-panthenol; polyethylene glycols; other commonly known pharmaceutically acceptable humectants, or their mixtures.

Thickener is selected from the group comprising: cellulose gums like hydroxypropyl methylcellulose (HPMC), hydroxypropyl cellulose (HPC), hydroxyethyl cellulose (HEC), methyl cellulose (MC), sodium carboxymethyl cellulose (NaCMC); synthetic polymers such as polyvinyl alcohol (PVA), polyacrylic acid (PAA) and its copolymers, polyvinylpyrrolidone (PVP); various gums like gum arabic, xanthan gum, guar gum, tragacanth; alginic acid and its salts like sodium alginate; mixtures of these substances, or other pharmaceutically acceptable thickeners.

Chelating agent is selected from the group comprising: sodium, or potassium salts of ethylenediaminotetraacetic (edetic) acid (EDTA); diethylenetriamine pentaacetic acid (DTPA); nitrilotriacetic acid (NTA); water soluble citrate salts like trisodium citrate dihydrate; mixtures of these substances, or other pharmaceutically acceptable chelating agents. Representative example of such chelating agent is disodium edetate dihydrate ($Na_2EDTA \bullet 2H_2O$).

Preservative is selected from the group comprising: parabens like methyl 4-hydroxybenzoate, ethyl 4-hydroxybenzoate, propyl 4-hydroxybenzoate, butyl 4-hydroxybenzoate; 4-chloro-*m*-cresol; triclosan; chlorobutanol; chlorhexidine and its salts; quaternary ammonium salts such as benzalkonium chloride or cetrimonium bromide; benzoic acid; sorbic acid; benzyl alcohol; 2-phenoxyethanol; dehydroacetic acid (3-acetyl-2-hydroxy-6-methyl-4*H*-pyran-4-one); mixtures of these substances, or other pharmaceutically acceptable preservatives.

Antioxidant is selected from the group comprising: ascorbic acid, its salts and esters like calcium ascorbate or ascorbyl palmitate; 2,6-di-tert-butyl-4-methylphenol (BHT); *tert*-butyl-anisole (BHA); propyl gallate; $\alpha$-tocopherol and its esters like $\alpha$-tocopheryl acetate; rosemary (*Rosmarinus officinalis* L.) extract; mixtures of these substances, or other pharmaceutically acceptable antioxidants.

As active pharmaceutical ingredient (API) of the composition from the present invention compound **1** or pharmaceutically acceptable salt or hydrate thereof were employed.

Since free phenolic OH groups of the compound **1** do act as acids, the corresponding salts with pharmaceutically acceptable, non-toxic bases can be prepared. Such salts do have certain advantages over free acid **1** due to eventually increased water solubility.

Examples of useful bases that can be employed for preparation of various pharmaceutically acceptable salts of compound **1** are selected from the group comprising: sodium hydroxide (NaOH), potassium hydroxide (KOH), magnesium hydroxide [$Mg(OH)_2$], calcium hydroxide [$Ca(OH)_2$], ammonium hydroxide ($NH_4OH$), tetramethyl/ethylammonium hydroxide [$R_4N^+OH^-$; R= $CH_3$, $C_2H_5$], choline hydroxide [$(CH_3)_3N(CH_2CH_2OH)OH$], other pharmaceutically acceptable bases, or mixtures of these substances in various molar ratios.

The syntheses of compound **1** and its pharmaceutically acceptable salts were performed by the procedure disclosed in our previous patent application; see literature reference 9.

<u>Preparation of the composition from the present invention</u>

The composition from the present invention involves all pharmaceutically useful final dosage forms as described above.

The technology for preparation of various final dosage forms is known to the person skilled in the art of pharmaceutical technology; for instance see literature reference 10:
10) J. Swarbrick, J. C. Boylan: Encyclopedia of pharmaceutical technology (1998-2001) M. Dekker, New York, USA.

The process for production of pharmaceutical composition according to the present invention is consisting **of** the following steps:

(i) addition of one or more pharmaceutical excipients into a mixing vessel;
(ii) addition of active pharmaceutical ingredient **1** into the homogenized base;
(iii) homogenization of thus obtained mixture;
(iv) optional addition of other required pharmaceutical excipients;
(v) final homogenization of the composition; and
(vi) filling of homogenized composition into the suitable dosage container; or
(vii) capsulation or tableting.

Final dosage form of powder including the inhalation powder is manufactured by homogenization of powderous ingredient 7,9-dihydroxy-3-(4,5,7-trihydroxy-2-oxo-2*H*-chromene-3-yl)-4*H*-furo[3,2-*c*]chromen-4-one (**1**) which serves as the active pharmaceutical ingredient (API) with one or more powderous excipients selected from the group comprising: filler, lubricant, and optionally, other pharmaceutical excipients.

Thus obtained powder can be granulated, by using one or more suitable diluents and binders, yielding dosage forms of granules. Diluents like purified water for wet granulation process are used on *quantum satis* (q.s.) principle.

Powders and granules can be compacted to give final dosage forms of tablets, or alternatively, filled into gelatin or various vegetable capsules furnishing final dosage form of capsules.

Liquid dosage forms such as oral solution, suspension, or syrup are prepared by dissolution of compound **1** in a suitable diluent like purified water, or mixtures of purified water with humectants and thickeners. Liquid formulations based on predominantly water have to be preserved against microbiological spoilage by addition of suitable preservative.

Topical dosage form of ointment is prepared by homogenization of fine powderous compound **1** into the hydrophilic or lipophilic ointment base. The former are, for instance, a mixture of solid and liquid polyethylene glycols (PEG), whilst the latter are various mixtures of waxes, plant oils, lanolin, etc.

Creams and lotions from the class of water-in-oil (W-O) or oil-in-water (O-W) emulsions are prepared by homogenization of compound **1** in the corresponding base emulsions.

There can be many other preparation technologies of the composition of the present invention in all mentioned and other possible final dosage forms, what is known to the person skilled in the art of pharmaceutical technology.

For demonstration, typical final dosage forms of the composition from this invention are given in Examples 4-10.

<u>Study of cytotoxicity of 7,9-dihydroxy-3-(4,5,7-trihydroxy-2-oxo-2*H*-chromene-3-yl)-4*H*-furo[3,2-*c*]chromen-4-one (**1**) via determination of maximal non-toxic concentration (MNC) and cytotoxic concentration ($CC_{50}$) in cell culture of murine origin</u>

Cytotoxicity of compound **1** was determined through the study of cell growth and viability by the methods of determination of maximal non-toxic concentration (MNC) and concentration required for cell viability by 50% ($CC_{50}$). Maximal non-toxic concentration (MNC) was defined as the highest concentration of the test substance **1**, which does not cause injury or death of the treated cells. Cytotoxic concentration at 50% ($CC_{50}$) was defined as the concentration of the test substance **1** at which 50% of the cells die as a result of toxicity of the test substance.

Cytotoxicity assays are performed to predict potential toxicity, using cultured cells which may be normal or transformed cells. These tests normally involve short term exposure of cultured cells to test substances, to detect how basal or specialized cell functions may be affected by the substance, prior to performing safety studies in whole organisms. Assessment parameters for cytotoxic effects include inhibition of cell proliferation, cell viability markers (metabolic and membrane), morphologic and intracellular differentiation markers and others; see literature reference 11:
11) P. O'Brien, J. Haskings: In vitro cytotoxicity assessment, Methods Mol. Biol. 356 (2006) 415-425.

Evaluation of cytotoxicity was an important part of the assessment of anti-asthmatic activity of compound **1** since its beneficial therapeutic effect at asthma and other inflammatory respiratory diseases should be selective with little or no effects on the metabolism of host cells. The study of cytotoxicity of compound **1** was performed on two types of cell lines of murine fibroblasts L20B and L929 from European collection of cell cultures (ECACC).

Cell viability was estimated by a modification of the 3-(4,5-dimethylthiazol-2-yl)-2,5-diphenyltetrazolium bromide (MTT) assay which is one of the most frequently used methods for measuring cell proliferation and cytotoxicity; see literature reference 12:
12) T. Mosmann: Rapid colorimetric assay for cellular growth and survival: Application to proliferation and cytotoxicity assays, J. Immunol. Methods 65 (1983) 55-63.

Cell viability was reported as the percentage (%) of viable cells in the wells treated with different concentrations of the test compound **1**, compared to the control cells untreated with the substance. Maximal non-toxic concentration (MNC) and cytotoxic concentration for 50% of cells ($CC_{50}$) were calculated from the constructed "dose-cellular survival" curve.

Dynamics of survival of murine cells treated with compound **1** at 48 h were presented in Table 1, whilst *in vitro* cytotoxicity data were summarized in Table 2.

Detailed experimental procedure was described in Example 1.

**Table 1.** Dynamics of survival of murine cell lines L20B and L929 treated with 7,9-dihydroxy-3-(4,5,7-trihydroxy-2-oxo-2*H*-chromene-3-yl)-4*H*-furo[3,2-*c*]chromen-4-one (**1**) at 48 h.

| No. | Concentration of 1 [mg/mL] | Cell survival [%] | |
|---|---|---|---|
| | | Cell culture L20B | Cell culture L929 |
| 1 | 0.0001 | 103.3 | 100.6 |
| 2 | 0.001 | 99.7 | 98.8 |
| 3 | 0.01 | 94.5 | 95.3 |
| 4 | 0.05 | 91.3 | 87.1 |
| 5 | 0.1 | 88.2 | 89.3 |
| 6 | 0.5 | 85.6 | 83.2 |
| 7 | 1 | 72.4 | 76.1 |
| 8 | 5 | 60.2 | 64.9 |
| 9 | 10 | 48.5 | 50.5 |

**Table 2.** Cytotoxic effect of 7,9-dihydroxy-3-(4,5,7-trihydroxy-2-oxo-2*H*-chromene-3-yl)-4*H*-furo[3,2-*c*]chromen-4-one (**1**) on murine fibroblasts cell lines L20B and L929 at 48 h.

| No. | Murine cell line | MNC [a] [mM] | $CC_{50}$ [b] [mM] |
|---|---|---|---|
| 1 | L20B | 0.001 | 9 |
| 2 | L929 | 0.0001 | 10 |
| [a] MNC= maximal non-toxic concentration.<br>[b] $CC_{50}$= cytotoxic concentration for 50% cells. | | | |

When microscopic observation of the morphology of the mono-layers were carried out at 48 h after the treatment with tested compound 1 in different concentration range (0.0001-10 mg/mL), typical cytopathology characterizing the toxic effect was not registered in both tested cell lines. We found some morphology changes of the cell lines in comparison with the cell control only in wells treated with the highest concentrations 10 mg/mL and 5 mg/mL, whose impact could

be due to the highest content of the solvent dimethyl sulfoxide (DMSO). To evaluate whether this effect is due to the toxicity of the tested compound **1** or DMSO, mortgaging control of medium DMEM or MEM supplemented with 2% FBS and DMSO. We conducted a test with the same concentration range as the test compound **1.** The results show that the concentration of DMSO in the lowest dilutions is extremely small and does not have a toxic effect on the tumor and normal cells.

In conclusion, the results showed that the tested compound **1** exhibit low cytotoxicity against both murine cell lines L20B and L929. These results were dose-dependent. According to MNC values, compound **1** at 48 h expressed 10x higher cytotoxicity against L929 cells than those on L20B cell line. According to $CC_{50}$ values, compound **1** at 48 h expressed the same cytotoxicity.

We concluded that these results additionally strongly support the initial hypothesis that compound **1** is essentially non-toxic, and thus can be safely used as the pharmaceutical active substance (API). Certainly, further toxicological studies have to be performed for its detail safety profile evaluation, but this is obviously not essential for demonstration of novelty and inventive step of this invention.

<u>Study of anti-inflammatory activity of 7,9-dihydroxy-3-(4,5,7-trihydroxy-2-oxo-2*H*-chromene-3-yl)-4*H*-furo[3,2-*c*]chromen-4-one (**1**) on *in vivo* model of croton oil-induced ear edema in mice</u>

Skin is responsible for the communication between an organism and the environment and is constantly subjected to exogenous stimuli. The main function of the skin is to protect the organism from environmental insults. Fulfilling its role, the skin is able to activate a defense mechanism aimed at pathogen elimination and tissue repair; see literature references 13 and 14:

13) E. Proksch, J. Brandner, J. Jensen: The skin: an indispensable barrier, Experimental Dermatology 12 (2008) 1063-1072.

14) C. De Benedictis, S. Joubeh, G. Zhang, M. Barria, R. Ghohestani: Immune functions of the skin, Clinics in Dermatology 5 (2001) 573-585.

Initiation of the defense response is characterized by the infiltration of neutrophils and the release of several proinflammatory mediators, which starts the inflammatory process. Acute inflammation is characterized by classical symptoms, such as heat, redness, swelling and pain. Edema (swelling) is therefore a good measure of inflammation and is useful for the quantification of skin inflammation induced by phlogistic agents such as croton oil. Croton oil-induced ear edema is a widely used method for studying the inflammatory process in skin, and for identifying anti-inflammatory agents that could be useful in the treatment of skin disorders; see literature reference 15:

15) L. De Young, J. Kheifets, S. Ballaron, J. Young: Edema and cell infiltration in the phorbol ester-treated mouse ear are temporally separate and can be differentially modulated by pharmacologic agents, Agents Actions 26 (1989) 335-341.

The presents study aims to detect the inflammatory/anti-inflammatory potential effects of compound **1** in a model of acute skin inflammation induced by local application of phlogistic agent croton oil in Balb/c mice. The technique for croton oil-induced ear edema in mice, originally described by Tubaro et *al.*, was followed, with modifications; see literature references 16 and 17:

16) A. Tubaro, P. Dri, G. Delbello, C. Zilli, R. Della Loggia: The croton oil ear test revisited, Agents Actions 17 (1986) 347-349.

17) G. Coruzzi, C. Pozzoli, M. Adami, D. Grandi, N. Guido, R. Smits, I. de Esch and R. Leurs: Strain-dependent effects of the histamine H4 receptor antagonist JNJ7777120 in a murine model of acute skin inflammation, Experimental Dermatology 21 (2011) 32-37.

Croton oil is a phlogistic agent extracted from *Croton tiglium L.*, *Euphorbiaceae*, which exhibits an irritant and vesiculant effect on the skin. Croton oil contains phorbol esters, predominantly 12-0-tetradecanoylphorbol-13-acetate (TPA). Topical application of croton oil or TPA promotes an acute inflammatory reaction characterized by vasodilatation, polymorphonuclear leukocyte infiltration to the tissue and edema formation. These changes are triggered by protein kinase C (PKC) activation, which promotes an increase in the activity of phospholipase $A_2$ ($PLA_2$). Activation of $PLA_2$ results in increased levels of arachidonic acid and its metabolites, such as prostaglandins and leukotrienes. Moreover, PKC also promotes the secretion and activation of several immune mediators such as cytokines and chemokines which increase and maintain the skin inflammatory response; see literature references 18-20:

18) M. Otuki, F. Vieira-Lima, Â. Malheiros, R. Yunes, J. Calixto. Topical antiinflammatory effects of the ether extract from Protium kleinii and $\alpha$-amyrin pentacyclic triterpene. European Journal of Pharmacology 507 (2005) 253-259.

19) X. Wang, M. Lan, H. Wu, Y. Shi, J. Lu, J. Ding, K. Wu, J. Jin, D. Fan. Direct effect of croton oil on intestinal

epithelial cells and colonic smooth muscle cells. World Journal of Gastroenterology 8 (2002) 103-107.
20) M. Denning. Epidermal keratinocytes: regulation of multiple cell phenotypes by multiple protein kinase C isoforms. International Journal of Biochemistry and Cell Biology 36 (2004) 1141-1146.

We extended the treatment up to 6 h to measure the peak of inflammation. Dexamethasone (1 mg/kg), represents the reference anti-inflammatory drug. Mice (total 40) were separated into 4 groups (n= 10). Each group was treated under different regimen:

Group 1: croton oil

| | |
|---|---|
| Right ear: | croton oil |
| Left ear: | acetone |

Group 2: dexamethasone + croton oil (after 30 min)

| | |
|---|---|
| Right ear: | dexamethasone + croton oil |
| Left ear: | 80% 2-hydroxypropyl-β-cyclodexin (2HPβCD) + 20% DMSO |

Group 3: compound **1** + croton oil (after 30 min)

| | |
|---|---|
| Right ear: | compound **1** + croton oil |
| Left ear: | 80% 2-hydroxypropyl-β-cyclodexin + 20% DMSO |

Group 4: untreated

| | |
|---|---|
| Right ear: | nothing |
| Left ear: | nothing |

Cutaneous inflammation was induced in conscious mice by topical application of croton oil (5% in acetone). Acetone was applied to the left ear, which served as a control; indeed, it has been previously demonstrated that acetone did not induce changes in the ear weight; see literature reference 21:
21) K. Hon, V. Lee, T. Leung, K. Lee, A. Chan, T. Fok, P. Leung. Corticosteroids are not present in a traditional Chinese medicine formulation for atopic dermatitis in children. Annals of the Academy of Medicine Singapore 35 (2006) 759-763.

Six hours after croton oil application, mice were euthanized by light ether anaesthesia, followed by cervical dislocation; both left (acetone) and right (croton oil in acetone) ears were removed, by cutting horizontally across the indentation at the base of the ear. For each mouse, the extent of the oedema was expressed as the difference in weight (Δ [mg]) between right (inflamed) and left (uninflamed) ear. The percentage increase in the oedema of the treated ear was calculated by the following formula:

$$\%\ \text{ear oedema} = \frac{\text{Wt. of tested ear} - \text{Wt. of control ear}}{\text{Wt. of control ear}} \bullet 100$$

Wt. = weight

Topical application of croton oil caused a significant inflammatory response in mouse skin, as determined by the increase in ear weight, when compared to the ear that received only vehicle (acetone) at 6 h. Effect of compound **1** and dexamethasone on croton oil-induced ear inflammation and induced a reduction of the ear oedema provoked by the local application of croton oil in Balb/c mice; approximately 60% maximal inhibition for dexamethasone and 40% for compound **1** was observed. Results are presented in Table 3.

**Table 3.** Results of the study of anti-inflammatory activity of 7,9-dihydroxy-3-(4,5,7-trihydroxy-2-oxo-2*H*-chromene-3-yl)-4*H*-furo[3,2-*c*]chromen-4-one (**1**) on *in vivo* model of croton oil-induced ear edema in mice; see Example 2.

| No. | Treatment regimen | Left ear m [g] | Right ear m [g] | Ear edema $\Delta$m [g][a] | %[b] |
|---|---|---|---|---|---|
| 1 | Group 1:[c]<br>Right ear: croton oil<br>Left ear: acetone | 0.0366 | 0.0888 | 0.0522 | 143.36 |
| 2 | Group 2:<br>Right ear: dexamethasone + croton oil[d]<br>Left ear: 80% 2HP$\beta$CD + 20% DMSO | 0.0360 | 0.0519 | 0.0159 | 44.2 |
| 3 | Group 3:<br>Right ear: **1** + croton oil[d]<br>Left ear: 80% 2HP$\beta$CD + 20% DMSO | 0.0344 | 0.0665 | 0.0321 | 92.98 |
| 4 | Group 4:[e]<br>Right ear: nothing<br>Left ear: nothing | 0.0361 | 0.0337 | -0.0025 | -6.75 |

[a] The ear oedema was expressed as the difference in weight ($\Delta$ [mg]) between right (inflamed) and left (uninflamed) ear.
[b] Percentage of increasing the ear edema at different treatment regimen.
[c] Positive control: Group 1: acetone (left ear), croton oil (right ear) .
[d] In the case of testing standard dexamethasone and compound **1**, croton oil was applied after 30 minutes.
[e] Negative control (no treatment).

Detailed experimental procedure is described in Example 2 and graphically shown in Figure 1.

Study of anti-asthmatic activity of 7,9-dihydroxy-3-(4,5,7-trihydroxy-2-oxo-2*H*-chromene-3-yl)-4*H*-furo[3,2-*c*]chromen-4-one (**1**) on *in vivo* model in mice

As explained earlier, asthma is characterized by airway inflammation, airway obstruction and bronchial hyper responsiveness; see literature reference 22 and 23:

22) N. Hanania: Targeting airway inflammation in asthma - current and future therapies. Chest 133 (2008) 989-998;
23) R. Djukanovic, W. Roche, J. Wilson, C. Beasley, O. Twentyman, P. Howarth, H. Holgate: Mucosal inflammation in asthma. Am. Rev. Respir Dis. 142 (1990) 434-457.

Asthma can be caused by various factors like allergens, drugs, respiratory infection, dust, cold air, exercise, emotions, occupational stimuli, chemicals, histamine, etc. Allergen provocation in asthma induces both an immediate asthmatic response (IAR) and a late-phase asthmatic response (LAR), both of which require long-term anti-inflammatory therapy; see literature reference 24:
24) T. Matsumoto, Y. Ashida, R. Tsukuda: Pharmacological modulation of immediate and late airways response and leukocyte infiltration in the guinea pig, J. Pharm. Exp. Ther. 269 (1994) 1236-1244.
In asthma patients, there is an accumulation of neutrophils, macrophages, activated mast cells, eosinophils, and T cells in the air spaces after antigen sensitization; see literature references 23 and 25:
25) M. Azzawi, B. Bradley, P. Jeffery, A. Frew, A. Wardlaw, G. Knowlwa, B. Assoufi, J. Collins, S. Durham and A. Kay: Identification of activated lymphocytes and eosinophils in bronchial biopsies in stable atopic asthma, Am. Rev. Respir. Dis. 142 (1990) 1407-1418.
There is now convincing evidence that cytokines secreted by T cells or other immune cells, such as interleukin-6 (IL-6), IL-10, IL-12, and interferon-g (IFN-g), in response to antigen stimulation play a role in lung inflammation and asthma. IL-6 serves as chemotactic factor for various leukocyte population and is an important proinflammatory factor. In patients with asthma, the levels of inflammatory cells, T-cells, and cytokines have been shown to be significantly elevated in bronchoalveolar lavage (BAL) fluids, suggesting a possible pathological role for these cells; see literature

references 26-28:

26) D. Dalton, K. Pitts-Mee: Multiple defects of immune cell function in mice with disputed interferon-g genes, Science (Wash DC) 259 (1993) 1739-1742.
27) K. Arai, F. Lee, A. Miyajima: Cytokines: Coordinators of immune and inflammatory response, Annu. Rev. Biochem. 59 (1990) 783-836.
28) C. Corrigan, A. Kay: T cells and eosinophils in the pathogenesis of asthma, Immunol. Today 13 (1992) 501-507.

Animal models have been used to elucidate asthma pathophysiology, and to identify and evaluate novel therapeutic targets. Allergen challenge models reproduce many features of clinical asthma and have been widely used by investigators; however, the majority involve acute allergen challenge procedures. It is recognised that asthma is a chronic inflammatory disease resulting from continued or intermittent allergen exposure, usually via inhalation, and there has been a recent focus on developing chronic allergen exposure models, predominantly in mice.

Although many different sensitisation and challenge protocols have been used, the basic model is consistent. Acute sensitisation protocols require multiple systemic administration of the allergen in the presence of an adjuvant. Adjuvants such as aluminium hydroxide ($AlOH_3$) are known to promote the development of the Th2 phenotype by the immune system when it is exposed to an antigen. Sensitisation solely via the airways has also been attempted using both ovalbumin (OVA) and house dust mite (HDM).

With the OVA models, after the sensitisation period, usually 14-21 days, the animal is challenged with the allergen via the airway, usually over a period of several days. Allergen may be inhaled as a nebulised formulation (aerosol), or administered by intranasal (i.n.) instillation of an aqueous formulation.

Model of lung eosinophilia in mice

The acute challenge mouse models reproduce many key features of clinical asthma, for example elevated levels of IgE, airway inflammation, mucus secretion, goblet cell hyperplasia, epithelial hypertrophy, airway hyperresponsiveness (AHR) to specific stimuli. Bronchoalveolar lavage (BAL) and histology studies indicate that the influx of inflammatory cells is dominated by eosinophils.

The study was peformed on female 8-week old Balb/c mice. The animals were kept under specific-pathogen-free (SPF) conditions with temperature control and HEPA system for breeding of laboratory animals. Experiments were performed during the light phase of the cycle. 40 mice (n= 10 /group) were separated into 4 groups:

Group 1: OVA only
Group 2: OVA + BECLOMETHASONE (1 mg/kg)
Group 3: OVA + Compound **1** (preventively; 2 mg/kg)
Group 4: OVA + Compound **1** (therapeutically; 2 mg/kg)

Each group of mice was sensitized on days 0 and 14 with 20 μg/mouse of OVA adsorbed on 2 mg alum. On days 20, 21 and 22 the animals were stimulated with 100 μg/mouse OVA intranasally in a final volume of 50 μL/mouse.

The first group (the positive control) was injected with 0.1 M phosphate-buffered saline (PBS) only.

The second and third groups were treated preventively with compound **1** (2 mg/kg) or beclomethasone (1 mg/kg) on days 18-22 via the airway.

The fourth group was treated therapeutically with compound **1** (2 mg/kg) on days 23-25 via the airway.

The detailed treatment schedule is presented in the Figure 2.

Preparation of bronchoalveolar lavage (BAL):

The experimental procedure was carried out as described in Example 3 according to literature reference 29:
29) F. Daubeuf, N. Frossard: Performing Bronchoalveolar Lavage in the Mouse, Curr. Protoc. Mouse Biol. 2 (2012) 167-175.

We examined the effect of compound **1** treatment in the cell response on OVA-induced airway inflammation. OVA (100 pg/50 μL of PBS) was introduced intranasally (i.n.) 6 days after the second intraperitoneal (i.p.) sensitization with OVA. The treatment with compound **1** (preventively and therapeutically), beclomethasone or PBS only was performed according the treatment schedule; see Figure 2.

Two days after the last i.n. challenge, BAL cells were collected, and differential cell counts were performed to identify the number of various infiltrating inflammatory cells, see Figures 3A, 3B.

Challenge of OVA induced eosinophil and macrophages infiltration into the lung, but blocked lymphocyte and neutrophil infiltration. The treatment with compound **1** (either preventively or therapeutically):

1. strongly suppressed the eosinophil infiltration into the lungs; but
2. stimulate macrophages, lymphocyte and neutrophil infiltration compared to PBS treated animals.

In contrast, standard anti-inflammatory active pharmaceutical ingredient beclomethasone, used as a standard in this experiment:

1. suppressed the eosinophil and neutrophil infiltration; but also,
2. induced moderate lymphocyte and macrophages response.

Lungs from the Balb/c mice from the test-groups were isolated and fixed in 10% formalin with 4 mL of the same solution introduced intratracheally prior the overnight fixation. Paraffin sections from the lungs were analyzed using a standard haematoxylin/eosin staining technique.

Histological analysis of sections of the lung tissue recovered from mice 2 days after a last treatment showed variation in the degree of asthma-like pathology, see Figure 4.

After allergen challenge, significantly more eosinophils, macrophages, lymphocytes and neutrophils were observed in challenged BALB/c mice. Massive cell infiltration was found in the lungs of the animals from OVA group treated with PBS only. Eosinophil and other inflammatory cell infiltration and differences in the lung histology between Beclomethasone-treated and compound **1** - treated (either preventively or therapeutically) animals were not observed, see Figure 4.

Compound **1** treatment reduces mucus production in the airway

Enhanced airway mucus production results from airway inflammation and is hallmark of allergic asthma. Thus, mechanisms that suppress immune responses in the lung could result in suppression of mucus production. Mucus production was measured 2 days after i.n. challenge in experimental mice that were treated with compound 1 or beclomethasone (as standard) and lung sections were stained with periodic acid-Schiff stain (PAS). Mucus production in the lung was quantitated immunohistologically by evaluation of mucus-positive epithelia.

PAS staining showed the increased mucus production in inflamed tissues of OVA group lungs treated with PBS only.

Mice that received compound **1** either therapeutically or preventively showed a significant reduction of mucin production, compared with mice that received no treatment; see Figure 5.

Similar reduction was observed in the mice group treated with standard antiinflammatory API beclomethasone.

These results indicate that compound **1** treatment that suppress the recruitment of immune cells to the lung also suppress mucus production, a pathological consequence of airway inflammation.

Experimental results are described in Example 3.

Histological and immunohistochemical studies of compound 1 effect on model mouses

Th2 cytokine and eotaxin production in allergic asthma: the Th2-associated cytokines IL-4, IL-5, IL-13 and eotaxin are central to the development of allergic asthma and OVA-sensitised mice exhibited high levels of interleukin 5 (IL-5). To investigate the treatment effect of compound **1**, immunohistology from serial lung sections was performed 2 days after i.n. challenge in experimental mice that were treated with the compound **1** or beclomethasone as standard antiinflammatory and anti-asthmatic API.

IL-5 staining showed the increased cytokine production in inflamed tissues of OVA group lungs treated with PBS only; see Figure 6. The staining pattern was similar for both compound **1** treated animal groups and no IL-5 expression was found, while moderate result was measured among beclomethasone-treated OVA-sensitised animals. Thus, the IL-5 expression from the airway epithelium appears to correlate with the observed lung histopathologies found in these animals.

Acute OVA provocation displayed evidence of an activated phenotype as characterized by CCL11 (eotaxin) expression in a subpopulation of lung cells. CCL11 staining showed stained positive for eotaxin cells of OVA group lungs treated with PBS only, see Figure 7, whereas levels were reduced to background following compound **1** and beclomethasone treatment. These results suggest that cells derived eotaxin are responsible for eosinophil accumulation in allergic lung inflammation found in the Figure 4.

Experimental results are described in Example 3.

Use of the composition from this invention

The composition of the present invention based on 7,9-dihydroxy-3-(4,5,7-trihydroxy-2-oxo-2*H*-chromene-3-yl)-4*H*-furo[3,2-*c*]chromen-4-one (**1**) as the active pharmaceutical ingredient, in its various final dosage forms, is used in the therapy of:

(i) asthma; and
(ii) other inflammatory respiratory diseases such as: allergic rhinitis and sinusitis, acute respiratory disease syndrome (ARDS), and chronic obstructive pulmonary disease (COPD).

Depending on the kind of final dosage form, the composition of the present invention can be administered: by inhalation, orally, topically, parenterally, or by any other suitable manner of therapeutic application.

The therapy involves one or more administrations per day of pharmaceutically effective doses from 0.1-15 mg/kg of body weight, what represents 7.5-1.125 mg of active compound **1** per average adult person of 75 kg body weight.

## Examples

General remarks

MTT method was performed spectrophotometrically on Epoch Microplate Spectrophotometer (BioTek Instruments, VT, USA).The microscope on which the histology was carried out was Nikon eclipse E100 (Nikon Instruments Europe B.V., Amstelveen, Netherlands).

Room temperature (r.t.) means the temperature interval of 20-25 °C.

Example 1. Study of cytotoxicity of 7,9-dihydroxy-3-(4,5,7-trihydroxy-2-oxo-2*H*-chromene-3-yl)-4*H*-furo[3,2-*c*]chromen-4-one (**1**) on *in vitro* model of murine cell cultures

Cytotoxicity of compound **1** was determined through the study of cell growth and viability by the methods of determination of maximal non-toxic concentration (MNC) and concentration required for cell viability by 50% ($CC_{50}$).

The compound **1** (Mw = 410.02) under study were first dissolved in DMSO (Sigma-Aldrich) to a concentration of 0.1 mol/L (0.1 M) as a stockade later diluted in cell growth medium Dulbecco's Modified Eagle Medium (DMEM; Sigma-Aldrich) with 2% heat inactivated fetal bovine serum.

The study of cytotoxicity of compound **1** was performed on two types of murine fibroblasts cell lines: L20B and L929 from European collection of cell cultures (ECACC). Cells were routinely maintained as adherent cell cultures in DMEM medium and containing 10% Fetal bovine serum (FBS; Gibco), 2 mM L-glutamine (Sigma-Aldrich), 100 U/mL penicillin G sodium (Sigma-Aldrich), 100 pg/mL streptomycin sulphate (Sigma-Aldrich) at 37 °C in a humidified air incubator containing 5% carbon dioxide ($CO_2$). Cultivation of the cells was continued with direct monitoring every two or three days using a phase contrast microscope. The cells are harvested in 1× trypsin/EDTA solution (Sigma-Aldrich). Cells were passages 1:3-1:5 at a density around 5-6•$10^4$ cells/mL, while the passage was re-suspended repeatedly.

Methods of determining cell growth, cell viability, maximal non-toxic concentration (MNC) and concentrations, required cell viability by 50% ($CC_{50}$): Cell viability was estimated by a modification of the MTT [3-(4,5-dimethylthiazol-2-yl)-2,5-diphenyltetrazolium bromide] assay; see literature reference 12. The MTT reduction assay is one of the most frequently used methods for measuring cell proliferation and cytotoxicity. The intensity of colour (measured spectrophotometrically) of the MTT formazan produced by living, metabolically active cells by measuring the activity of succinate dehydrogenase, mostly located in mitochondria was proportional to the number of live cells present. MTT was a yellow water-soluble tetrazolium dye that was reduced by live, but not dead cells to a purple formazan product that was insoluble in aqueous solutions.

Each cell line was plated at an appropriate density (7.5•$10^3$ cells/well for L20B cell culture and 4•$10^4$ cells/well for L929 cell culture) in 96-well plates (Costar Corning; USA) for 24 h. When the adherent cells were stuck to the plastic, the supernatant was decanted and 50 μL of previously prepared diluted solutions of tested compound **1** were added. The well was completed with culture medium (150 pL) to a final volume of 200 μL. The plate was incubated for 48 h at 37°C and 5% content of $CO_2$. The cells grown in the medium without compound **1** served as a negative control. After 48 h incubation, the medium was replaced with MTT (Sigma-Aldrich) and dissolved at a final concentration of 5 mg/mL in serum-free medium, for further 3 h incubation. Then, the MTT-formazan product was solubilized in a mixture of ethanol:DMSO (1:1, V/V), and the optical density was measured at a test wavelength of 540 nm in microplate reader (Bio-Tek Instruments) using wells without sample containing cells as blanks. Each experiment was carried out in triplicate. Cell viability was reported as the % of viable cells in the wells treated with different concentrations of the tested compound compared to the control untreated cells. MNC and $CC_{50}$ were calculated from the constructed "dose - cellular survival" curve.

Dynamics of survival of murine cells treated with compound **1** at 48 h were presented in Table 1.

Both MNC and $CC_{50}$ values were evaluated simultaneously by morphological and by cell survival criteria. The

results obtained in both cells lines are given in Table 2.

Example 2. Study of anti-inflammatory activity of 7,9-dihydroxy-3-(4,5,7-trihydroxy-2-oxo-2*H*-chromene-3-yl)-4*H*-furo[3,2-*c*]chromen-4-one (**1**) on *in vivo* model of croton oil-induced ear edema in mice

The presents study aims to detect the inflammatory/anti-inflammatory potential effects of compound **1** in a model of acute skin inflammation induced by local application of phlogistic agent croton oil in Balb/c mice. The technique for croton oil-induced ear edema in mice, originally described by Tubaro et *al.*, was followed, with modifications; see literature references 16 and 17.

We extended the treatment up to 6 h to measure the peak of inflammation. Dexamethasone (1 mg/kg), represents the reference anti-inflammatory drug. Mice (total 40) were separated into 4 groups (n= 10). Each group was treated under different regimen:

Group 1: croton oil

| | |
|---|---|
| Right ear: | croton oil |
| Left ear: | acetone |

Group 2: dexamethasone + croton Oil (after 30 min)

| | |
|---|---|
| Right ear: | dexamethasone + croton oil |
| Left ear: | 80% 2-hydroxypropyl-β-cyclodexin + 20% DMSO |

Group 3: compound **1** + croton Oil (after 30 min)

| | |
|---|---|
| Right ear: | compound **1** + croton oil |
| Left ear: | 80% 2-hydroxypropyl-β-cyclodexin + 20% DMSO |

Group 4: Untreated

| | |
|---|---|
| Right ear: | nothing |
| Left ear: | nothing |

Cutaneous inflammation was induced in conscious mice by topical application of croton oil (5% in acetone). Acetone was applied to the left ear, which served as a control; indeed, it has been previously demonstrated that acetone did not induce changes in the ear weight; see literature reference 21.

In preliminary experiments, the effect of subcutaneous (*s.c.*) administration of saline, 100% DMSO or 20% 2-hydroxypropyl-β-cyclodextrin was investigated to check the vehicle effects on ear inflammation. Because DMSO significantly reduced croton oil-induced oedema, compound **1** (2 mg/kg) was dissolved in a vehicle containing 20% DMSO and 80% 2-hydroxypropyl-β-cyclodextrin, in amounts which did not change ear swelling *per se.* The positive control dexamethasone (1 mg/kg) was administered 30 min before the topic application of croton oil.

Six hours after croton oil application, mice were euthanized by light ether anaesthesia, followed by cervical dislocation; both left (acetone) and right (croton oil in acetone) ears were removed, by cutting horizontally across the indentation at the base of the ear. For each mouse, the extent of the oedema was expressed as the difference in weight (Δ [mg]) between right (inflamed) and left (uninflamed) ear. The percentage increase in the oedema of the treated ear was calculated by the following formula:

$$\%\ \text{ear oedema} = \frac{\text{Wt. of tested ear} - \text{Wt. of control ear}}{\text{Wt. of control ear}} \bullet 100$$

Wt. = weight

The statistical significance between the groups was assessed by one-way analysis of variance (ANOVA) followed by a *post hoc* Tukey test. The accepted level of significance for the test was P < 0.05. All tests were carried out

using GraphPad Software (San Diego, CA, USA). Topical application of croton oil caused a significant inflammatory response in mouse skin, as determined by the increase in ear weight, when compared to the ear that received only vehicle (acetone) at 6 h. Effect of compound **1** and dexamethasone on croton oil-induced ear inflammation and induced a reduction of the ear oedema provoked by the local application of croton oil in Balb/c mice; approximately 60% maximal inhibition for dexamethasone and 40% for compound **1.** Results are presented in Table 3 and in Figure 1.

Example 3. Study of anti-asthmatic activity of 7,9-dihydroxy-3-(4,5,7-trihydroxy-2-oxo-2*H*-chromene-3-yl)-4*H*-furo[3,2-*c*]chromen-4-one (**1**) on in *vivo* model in mice

Model of lung eosinophilia in mice

The acute challenge mouse models reproduce many key features of clinical asthma, for example elevated levels of IgE, airway inflammation, mucus secretion, goblet cell hyperplasia, epithelial hypertrophy, airway hyperresponsiveness (AHR) to specific stimuli.

Bronchoalveolar lavage (BAL) and histology studies indicate that the influx of inflammatory cells is dominated by eosinophils.

OVA and beclomethasone were purchased from Sigma-Aldrich (St Louis, MO, USA). Aluminium hydroxide [alum; $Al(OH)_3$] was purchased from BulBio (Sofia, Bulgaria). Compound **1** (Mw = 410.02) was synthesized by the process described in literature reference 9.

Female 8-week old Balb/c mice were obtained from Harlan Farm (Blackthorn, UK). The animals were kept under specific-pathogen-free (SPF) conditions with temperature control and HEPA system for breeding of laboratory animals. The manipulations were approved by the Animal Care Commission at the Institute of Microbiology in accordance with the International regulations (EU Directive 2010/63/EU).

Experiments were performed during the light phase of the cycle. The animals were allowed to adapt to the laboratory for at least 1h before testing and were used only once. 40 mice (n= 10 /group) were separated into 4 groups:

Group 1: OVA only
Group 2: OVA + beclomethasone (1 mg/kg)
Group 3: OVA + compound **1** (preventively; 2 mg/kg)
Group 4: OVA + compound **1** (therapeutically; 2 mg/kg)

Each group of mice was sensitized on days 0 and 14 with 20 μg/mouse of OVA adsorbed on 2 mg alum. On days 20, 21 and 22 the animals were stimulated with 100 μg/mouse OVA intranasally in a final volume of 50 μL/mouse.

The first group (the positive control) was injected with 0.1 M phosphate-buffered saline (PBS) only.

The second and third groups were treated preventively with compound **1** (2 mg/kg) or beclomethasone (1 mg/kg) on days 18-22 via the airway.

The fourth group was treated therapeutically with compound **1** (2 mg/kg) on days 23-25 via the airway.

The detailed treatment schedule is presented in the Figure 2.

Preparation of bronchoalveolar lavage (BAL)

The experimental procedure was carried out as described in literature reference 22. The procedure is as follows:

(1) using scissors, a small incision in the neck skin on the trachea was made and the skin upwards was opened to expose the salivary glands; the trachea was exposed;
(2) a small semi-excision of the trachea was made to allow a 21-G lavage tube to pass into the trachea;
(3) collection of the lavage fluid - 0.5 mL of sterile PBS was injected into the lung. The saline from the first lavage was aspirate; the procedure was repeated 5 times;
(4) all lavages were pooled and were centrifuge for 5 min. at 300 × g/4 °C to pellet the cells; the supernatant was discarded;
(5) the pellet was re-suspended in 1.5 mL ice cold PBS and centrifuge for 5 min. at 300 × g/4 °C; the supernatant was discarded;
(6) the pellet was re-suspended in 1.0 mL ice cold PBS and the cells were counted;
(7) 50 000 cells were placed on the slide for cytospin;
(8) the cytrospin was performed for 10 min at 700 rpm; and
(9) the slides were air-dried and proceeded to cell staining.

Cell Staining using Diff Quick staining kit (Polysciences Europe GmbH):

(1) fix in Solution A (fixative);
(2) dip 5 times in Solution C (red);
(3) dip 5 times in Solution B (blue);
(4) rinse the slides in distilled water;
(5) air-drying of the slides;
(6) mounted the cover slides; and then
(7) 400 cells were counted under light microscopy.

Histology staining was then performed as follows:

The lungs from all animals were fixed in 10% formalin (pH 7.4). Once fixed, tissue is processed as follows:

(1) 70% ethanol for 1 hour;
(2) 80% ethanol for 1 hour;
(3) 95% ethanol for 1 hour;
(4) first absolute ethanol for 1 hour;
(5) second absolute ethanol 1 hour;
(6) first clearing agent (xylene) for 1 hour;
(7) second clearing agent (xylene) for 1 hour;
(8) first wax at 58 °C for 1 hour;
(9) second wax at 58 °C for 1 hour; and
(10) embedding tissues in paraffin blocks.

Tissues were sectioned using a microtome at 5 $\mu$m slices.

Hematoxylin/Eosin staining:

(1) deparaffinize slides in 2 changes of xylene, 5 min. each;
(2) transfer slides to 100% alcohol, for 2 changes, 3 min. each;
(3) transfer once through 95% alcohol for 3 min;
(4) transfer to 80% and 70% alcohols respectively for 1 min. each;
(5) washing in distilled water for 2 min;
(6) stain in hematoxylin solution, Gill.2 (Sigma-Aldrich) for 3 min;
(7) tap water wash;
(8) blue in Scott's Tap water for 30 sec;
(9) tap water wash;
(10) acidified Eosin Y Solution (Sigma-Aldrich) for 1 min;
(11) tap water wash;
(12) dehydrate the tissue slides through 4 changes of alcohol (70%, 80%, 95%, 95%) for 1 min. each, and then, two times in 100% alcohol for 3 min. each; and
(13) clear the tissue slides in xylene for 5 min and coverslip using mounting solution.

PAS Staining:

(1) deparaffinize slides in 2 changes of xylene, 5 min. each;
(2) transfer slides to 100% alcohol, for 2 changes, 3 min. each;
(3) transfer once through 95% for 3 min;
(4) transfer to 80% and 70% alcohols respectively for 1 min. each;
(5) rinse quickly with distilled water;
(6) hydrolyse with 1% periodic acid solution for 10 min;
(7) rinse with tap water for 10 min;
(8) rinse with distilled water two times for 2 min. each;
(9) stain with Schiff's reagent (room temperature) for 15 min;
(10) rinse with warm tap water for 5 min;

(11) rinse quickly with distilled water;
(12) counterstain with hematoxylin solution, Gill.2 for 3 min;
(13) blue in flowing tap water for 10-15 min;
(14) dehydrate the tissue slides through 4 changes of alcohol (70%, 80%, 95%, 95%) for 1 min. each, and then, two times in 100% alcohol for 3 min. each;
(15) clear the tissue slides in xylene for 5 min. and coverslip using mounting solution.

The effect of compound **1** treatment in the cell response on OVA-induced airway inflammation was examined. OVA (100 $\mu$g/50 $\mu$L of PBS) was introduced intranasally (i.n.), 6 days after the second intraperitoneal (i.p.) sensitization with OVA. The treatment with compound **1** (preventively and therapeutically), beclomethasone or PBS only was performed according the treatment schedule; see Figure 2.
Two days after the last i.n. challenge, BAL cells were collected, and differential cell counts were performed to identify the number of various infiltrating inflammatory cells, see Figures 3A, 3B.
Challenge of OVA induced eosinophil and macrophages infiltration into the lung, but blocked lymphocyte and neutrophil infiltration. The treatment with compound **1** (either preventively or therapeutically) strongly suppressed the eosinophil infiltration into the lungs, but stimulate macrophages, lymphocyte and neutrophil infiltration compared to PBS treated animals.
In contrast, standard anti-inflammatory active pharmaceutical ingredient beclomethasone, used as a standard in this experiment suppressed the eosinophil and neutrophil infiltration, but also induced moderate lymphocyte and macrophages response.
Lungs from the Balb/c mice from the test-groups were isolated and fixed in 10% formalin with 4 mL of the same solution introduced intratracheally prior the overnight fixation. Paraffin sections from the lungs were analyzed using a standard haematoxylin/eosin staining technique.
Histological analysis of sections of the lung tissue recovered from mice 2 days after a last treatment showed variation in the degree of asthma-like pathology, see Figure 4.
After allergen challenge, significantly more eosinophils, macrophages, lymphocytes and neutrophils were observed in challenged BALB/c mice. Massive cell infiltration was found in the lungs of the animals from OVA group treated with PBS only. Eosinophil and other inflammatory cell infiltration and differences in the lung histology between beclomethasone-treated and compound **1**-treated (either preventively or therapeutically) animals were not observed, see Figure 4.

Compound **1** treatment reduces mucus production in the airway

Mucus production was measured 2 days after i.n. challenge in experimental mice that were treated with compound **1** or beclomethasone (as standard) and lung sections were stained with PAS. Mucus production in the lung was quantitated immunohistologically by evaluation of mucus-positive epithelia.
PAS staining showed the increased mucus production in inflamed tissues of OVA group lungs treated with PBS only. Mice that received compound **1** either therapeutically or preventively showed a significant reduction of mucin production, compared with mice that received no treatment; see Figure 5.
Similar reduction was observed in the mice group treated with standard antiinflammatory API beclomethasone.
These results indicate that compound **1** treatment that suppress the recruitment of immune cells to the lung also suppress mucus production, a pathological consequence of airway inflammation.

Histological and immunohistochemical studies of compound **1** effect on model mouses

Protocol for Chromogenic IHC Staining of Paraffin-embedded Tissue Sections (IL5 and CCL11)

Materials:

(1) Wash Buffer: T-PBS (0.1M, pH 7.4, 0.05% Tween 20);
(2) Blocking/Incubation Buffer: 1% bovine serum albumin, 0.1% Cold Fish Skin Gelatin, 0.5% Triton® X-100, 0.1 M PBS (pH 7.4);
(3) Peroxidase Blocking Solution (3% $H_2O_2$ in water);
(4) EDTA Antigen Retrieval Buffer (1mM EDTA, 0.05% Tween 20, pH 8.0);
(5) Primary antibodies:

- Rabbit anti-mouse IL-5 (Cat.# ABIN446956; Antibodies-Online GmbH,Schloss-Rahe-Str. 1552072 Aachen,Germany);
- Rat anti-mouse CCL11 (Cat.# MAB420; R&D Systems, Inc., 614 McKinley Place NE, Minneapolis, MN 55413);

(6) Secondary antibodies:

- Goat anti-rabbit IgG (H+L)-HRP (cat.# 31466; Thermo Fisher Scientific, 168 Third Avenue Waltham, MA USA 02451);
- Goat anti-rat IgG (H+L) - HRP (cat.# 62-9520; Thermo Fisher Scientific, 168 Third Avenue Waltham, MA USA 02451);

(7) Pierce DAB Substrate Kit (DAB (10X), 25 mL; Stable Peroxide Buffer, 250 mL) (Catalog # 34002; Thermo Fisher Scientific, 168 Third Avenue Waltham, MA USA 02451);
(8) Hematoxylin Counterstain (Cat # GHS216; Sigma-Aldrich Chemie GmbH, Eschenstrasse 5, 82024 Taufkirchen, Germany);
(9) Vitro-Clud 500 Mounting Medium (Catalog # A20500; Deltalab; Plz. Verneda 1, Pol Ind La Llana 08191 Rubí Barcelona Spain).

Procedure:

(1) Tissue was rehydrated before performing the staining protocol:

(1.1.) Immerse the slides in xylene (mixed isomers) 2 times for 10 minutes each.
(1.2.) Immerse the slides in 100% alcohol 2 times for 10 minutes each.
(1.3.) Immerse the slides in 95% alcohol for 5 minutes.
(1.4.) Immerse the slides in 80% alcohol for 5 minutes.
(1.5.) Immerse the slides in 70% alcohol for 5 minutes.
(1.6.) Rinse the slides with deionized $H_2O$.
(1.7.) Rehydrate the slides with wash buffer for 10 minutes. Drain the excess wash buffer.

(2) Antigen retrieval protocol - Adjust the power level of the microwave to 300 W. Pre-heat the EDTA buffer to 95-100 °C. Immerse slides in the staining dish and turn on the oven for 2-5 min or until the solution stars boil. Repeat two times. Place the staining dish at room temperature and allow slides to cool for 20-30 min. Wash slides three times for 2 min. with T-PBS.
(3) To quench endogenous peroxidase activity, incubate the slides with 1-3 drops peroxidase blocking reagent (3% $H_2O_2$ in water) for 15 minutes.
(4) Rinse the sample, then gently wash in wash buffer for 5 minutes.
(5) To reduce non-specific hydrophobic interactions between the primary antibodies and the tissue, block the section with 1ml per slide with blocking reagent for 30 minutes. Drain the slides and wipe away any excess blocking reagent before proceeding to the next step. Do not rinse.
(6) Incubate the slides with primary antibodies in Incubation buffer following manufacturer's recommendations: Rabbit anti-mouse IL-5 - 1:50; Rat anti-mouse CCL11 - 15 μg/mL. The slides were incubated overnight at 2-8 °C. All IHC experiments include a negative control using the incubation buffer with no primary antibody to identify non-specific staining of the secondary reagents.
(7) Rinse the samples with wash buffer. Wash 3 times with wash buffer for 5 minutes each, and drain the slides.
(8) Incubate the samples with 1 mL per slide of secondary antibodies in Incubation Buffer following manufacturer's recommendations: Goat anti-rabbit IgG (H+L)-HRP - 1:250; Goat anti-rat IgG (H+L) - HRP - 1:500. The slides were incubated for 3 hours/room temperature.
(9) Rinse with wash buffer 3 times for 10 minutes each and drain the slides.
(10) Calculate the required working volume of DAB Chromogen Solution. Add 1 mL DAB Chromogen Solution to cover the entire tissue section and incubate for 10 minutes. Rinse the sample with wash buffer 3 times for 10 minutes each.
(11) Rinse in deionized water and drain the slides.
(12) Stained tissue can be mounted either without nuclear counterstaining or counterstained with nuclear counterstain hematoxylin for better visualization of the tissue morphology.
(13) Cover stained tissue with a coverslip of an appropriate size, place slides vertically on filter paper or a towel to

drain excess mounting medium, and allow them to dry.
(14) Visualize staining of tissue under a microscope Nikon eclipse E 100 (Nikon Instruments Europe B.V., Laan van Kronenburg 2, 1183AS Amstelveen, Netherlands).

Results from this part of the study are presented in Figures 6 and 7.

Example 4. Preparation of the composition from the invention in the dosage form of tablets with 100 mg of 7,9-dihydroxy-3-(4,5,7-trihydroxy-2-oxo-2*H*-chromene-3-yl)-4*H*-furo[3,2-c]chromen-4-one (**1**) per tablet

Composition (for 1000 g of tablets):

(1) 100.00 g (10.00%) compound **1**
(2) 30.00 g (3.00%) cross-linked polyvinylpyrrolidone
(3) 12.00 g (1.20%) colloidal silicon dioxide Aerosil 200
(4) 858.00 g (85.80%) lactose monohydrate
(5) q.s. purified water

Preparation: Powderous compound **1** was homogenized with lactose monohydrate in V-blender for 10 minutes, subsequently granulated with aqueous solution of hydroxypropyl cellulose. The wet mass was forced through a sieve and granulate dried in an oven. After drying, granulate was mixed with polyvinylpyrrolidone and colloidal silicon dioxide, and homogenized in V-blender for 10 minutes. The resulting mixture was pressed into tablet cores. Tablet content: 100 mg ± 10% of compound **1** per tablet.

Example 5. Preparation of the composition from the invention in the dosage form of capsules with 50 mg 7,9-dihydroxy-3-(4,5,7-trihydroxy-2-oxo-2*H*-chromene-3-yl)-4*H*-furo[3,2-c]chromen-4-one (**1**) per capsule

Composition (for 1000 g of homogeneous mixture for capsules filling):

(1) 123.00 g (12.30%) compound **1**
(2) 15.00 g (1.50%) magnesium stearate
(3) 862.00 g (86.20%) maltodextrin Glucidex 1

Preparation: Previously weighted ingredients (1-3) were deagglomerated and homogenized in V-blender for 20 minutes. Thus obtained homogeneous powder was filled into transparent vegetable (HPMC) capsules of size 0 using manual capsule filling machine. Obtained capsules were of average weight of 500 mg. Capsule content: 50 mg ± 15% of compound **1** per capsule.

Example 6. Preparation of the composition from the invention in the dosage form of nebuliser solution with 0.01% of 7,9-dihydroxy-3-(4,5,7-trihydroxy-2-oxo-2*H*-chromene-3-yl)-4*H*-furo[3,2-c]chromen-4-one (**1**)

Composition (for 100 g of nebuliser solution):

(1) 0.01 g (0.01%) compound **1**
(2) 0.01 g (0.01%) disodium edetate dihydrate
(3) 0.10 g (0.10%) sodium dihydrogenphosphate
(4) 0.05 g (0.05%) sodium hydrogenphosphate
(5) 0.90 g (0.90%) sodium chloride
(6) 1.00 g (1.00%) glycerol
(7) 97.93 g (97.93%) sterile water

Preparation: Sodium chloride, sodium phosphates, and disodium edetate dihydrate were added to a mixture of water and glycerol, and dissolved by stirring at room temperature for 15 minutes. Then, compound **1** was added and homogenized by stirring at r.t. for 15 minutes. Thus obtained solution was filtered through:

(1) 1.2 μm filter to eliminate traces of mechanical impurities;
(2) 0.2 μm filter to reduce bioburden; and
(3) 0.1 μm sterile filter.

The product was in the form of almost odourless to pale yellow liquid. Analysis showed the content of 0.1 mg/mL ± 10% of compound **1.** The solution was filled into 20 mL plastic (PE-HD) bottles with inhalation device suitable for administration via inhalation of fine mist.

Example 7. Preparation of the composition from the invention in the dosage form of dry powder for inhalation with 10% of 7,9-dihydroxy-3-(4,5,7-trihydroxy-2-oxo-2*H*-chromene-3-yl)-4*H*-furo[3,2-c]chromen-4-one (**1**)

Composition (for 100 g of inhaling powder):

(1) 10.00 g (10.00%) compound **1**, micronized
(2) 90.00 g (90.00%) lactose monohydrate, micronized

Preparation: Previously weighted ingredients (1,2) were de-agglomerated and homogenized in V-blender for 5 minutes. The product was in the form of pale yellowish to pale brown fine free-flowing powder. The product was filled into PE-HD container with high pressure spraying device.
Analysis showed the content of 100 mg/g ± 10% of compound **1**.

Example 8. Preparation of the composition from the invention in the dosage form of injection with 0.1% of 7,9-dihydroxy-3-(4,5,7-trihydroxy-2-oxo-2*H*-chromene-3-yl)-4*H*-furo[3,2-*c*]chromen-4-one (**1**) monosodium salt

Compound **1** monosodium salt was prepared according to the procedure decribed in literature reference 9.
Composition (for 100 g of injection solution):

(1) 0.10 g (0.10%) compound **1** monosodium salt
(2) 0.80 g (0.80%) sodium chloride
(3) 0.10 g (0.10%) sodium dihydrogenphosphate
(4) 0.05 g (0.05%) sodium hydrogenphosphate
(5) 0.01 g (0.01%) disodium edetate dihydrate
(6) 98.94 g (98.94%) sterile water
(7) q.s. 10% hydrochloric acid and/or 10% sodium hydroxide

Preparation: Sodium chloride, sodium phosphates, disodium edetate dihydrate were added to sterile water, and dissolved by stirring at room temperature for 15 minutes. Then, compound **1** monosodium salt was added and homogenized by stirring at r.t. for 10 minutes. The pH value of the solution was corrected to the value 7.2-7.4 by addition of 10% hydrochloric acid and/or 10% sodium hydroxide solution. Thus obtained solution was filtered through:

(1) 1.2 μm filter to remove eventual traces of mechanical impurities;
(2) 0.2 μm filter to reduce bioburden; and
(3) 0.1 μm sterile filter.

The product was in the form of pale yellow almost odourless liquid. Analysis showed the content of 1.0 mg/mL ± 10% of compound **1**. The solution was filled into 20 mL plastic (PE-HD) bottles with spraying device or alternatively into containers pulmonal aerosols suitable as inhalation device.

Example 9. Preparation of the composition from the invention in the dosage form of ointment with 3% w/w of 7,9-dihydroxy-3-(4,5,7-trihydroxy-2-oxo-2*H*-chromene-3-yl)-4*H*-furo[3,2-*c*]chromen-4-one (**1**)

Composition (for 100 g of ointment):

(1) 3.00 g (3.00%) compound **1**
(2) 3.00 g (3.00%) lanolin alcohol
(3) 2.00 g (2.00%) cetostearyl alcohol
(4) 20.00 g (20.00%) mineral oil, heavy
(5) 72.00 g (72.00%) petroleum jelly

Preparation: Mixture of petroleum jelly, heavy mineral oil, lanolin and cetostearyl alcohol was melted at 50-55 °C with stirring until clear pale yellow liquid is obtained. Then, compound **1** was added, and homogenized by stirring with gradual cooling from 50 °C to 30-35 °C during 30 min, and filled at this temperature into 30 mL jars. Thus obtained

ointment was in the form of pale yellow occlusive grease of slight odour after lanolin.

Analysis showed the content of 2.70-3.30% of compound **1** (3.00 g compound **1**/100g of ointment; ± 10%).

Example 10. Preparation of the composition from the invention in the dosage form of cream with 1% w/w of 7,9-dihydroxy-3-(4,5,7-trihydroxy-2-oxo-2*H*-chromene-3-yl)-4*H*-furo[3,2-*c*]chromen-4-one (**1**)

Composition (for 100 g of cream):

(1) 1.00 g (1.00%) compound **1**
(2) 5.00 g (5.00%) 1,2-propylene glycol
(3) 4.00 g (4.00%) cetyl alcohol
(4) 4.00 g (4.00%) glyceryl monostearate
(5) 5.00 g (5.00%) polysorbate 60
(6) 5.00 g (5.00%) hexadecyl palmitate
(7) 10.00 g (10.00%) isopropyl myristate
(8) 0.25 g (0.25%) citric acid, anhydrous
(9) 0.50 g (0.50%) ammonium hydrogencitrate
(10) 0.20 g (0.20%) methyl 4-hydroxybenzoate (methylparaben)
(11) 0.10 g (0.10%) propyl 4-hydroxybenzoate (propylparaben)
(12) 64.95 g (64.95%) purified water

Preparation: Oil phase was prepared by melting of isopropyl myristate, hexadecyl palmitate, polysorbate 60, glyceryl monostearate, and cetyl alcohol at 70-75 °C. Aqueous phase was prepared by dissolving methyl and propyl parabens, citric acid, and ammonium hydrogencitrate in a mixture of purified water and 1,2-propylene glycol at 45-50 °C with stirring. Thus obtained solution was heated to 70-75 °C. Then, hot aqueous phase (70-75 °C) was added dropwise with intensive stirring to the oil phase (70-75 °C) during 30 minutes. The mixture was stirred at 70-60 °C during 30 minutes and at 60-50 °C during additional 30 minutes. Thus obtained emulsion was further homogenized by stirring at 50 °C down to 25 °C during 1 h. The product was filled into suitable containers, e.g. jars or tubes. The product was in the form of almost odourless, light oil-in-water (O-W) cream.

Analysis showed the content of 0.90-1.10% of compound **1** (1.00 g compound **1**/100g of ointment; ± 10%).

Conclusion:

The composition from the present invention based on 7,9-dihydroxy-3-(4,5,7-trihydroxy-2-oxo-2*H*-chromene-3-yl)-4*H*-furo[3,2-*c*]chromen-4-one (**1**) its pharmaceutically acceptable salts or hydrates thereof as the active pharmaceutical ingredient and one or more pharmaceutical excipients, required to yield final dosage forms suitable for therapeutic administration, clearly exhibits a profound:

(1) anti-inflammatory action; as demonstrated by *in vivo* model of croton oil-induced ear edema in mice; and
(2) anti-asthmatic activity; as proved by *in vivo* study in mice wherein:

- (a) strongly suppressed the eosinophil infiltration into the lungs; but
- (b) stimulate macrophages, lymphocyte and neutrophil infiltration compared to the control (PBS treated) animals; and
- (c) showed a significant reduction of mucin production, compared with mice that received no treatment; see Figure 5. Similar reduction was observed in the mice group treated with standard anti-inflammatory API beclomethasone.

These results indicate that:

1. the treatment with the compositon of the present invention suppresses the recruitment of immune cells to the lung and also a mucus production, which is a pathological consequence of airway inflammation; and
2. that the composition of the present invention is an effective anti-asthmatic agent and intiinflammatory agent for treatment of athma and other inflammatory respiratory diseases.

**Industrial Applicability**

Compound 7,9-dihydroxy-3-(4,5,7-trihydroxy-2-oxo-2*H*-chromene-3-yl)-4*H*-furo[3,2-*c*]chromen-4-one (**1**) or a

pharmaceutically acceptable salt or hydrate thereof is used as active pharmaceutical ingredient (API) for production the composition used in manufacturing of medicament for treatment of asthma and other inflammatory respiratory diseases. Thus the industrial applicability of this invention is obvious.

## List of abbreviations

| | |
|---|---|
| AHR - | airway hyperresponsiveness |
| alum - | aluminium hydroxide [$Al(OH)_3$] |
| API - | active pharmaceutical ingredient |
| BAL - | bronchoalveolar lavage |
| Balb/c - | an albino, laboratory bred strain of the house mice |
| BECLO - | beclomethasone (a well-known steroidal anti-inflammatory API) |
| $CC_{50}$ - | concentration required for cell viability of 50% |
| DEXA - | dexamethasone (a well-known steroidal anti-inflammatory API) |
| DMEM - | Dulbecco's Modified Eagle Medium |
| DMSO - | dimethylsulfoxide (solvent) |
| ECACC - | European collection of cell cultures |
| EDTA - | ethylenediamine tetraacetic acid (chelating agent) |
| FBS - | fetal bovine serum |
| HDM - | house dust mite |
| HEPA - | high-efficacy particulate air (filter) |
| 2HPβCD - | 2-hydroxypropyl-β-cyclodextrin |
| IAR - | immediate asthmatic response |
| IFN-g - | interferon-g |
| IL - | interleukin |
| LAR - | late-phase asthmatic response |
| mM - | milimolar concentration (molarity) [mmol/L] |
| M - | molar concentration (molarity) [mol/L] |
| MEM - | Minimum essential medium eagle (media) |
| MNC - | maximal non-toxic concentration |
| MTT - | [3-(4,5-dimethylthiazol-2-yl)-2,5-diphenyltetrazolium bromide] |
| OVA - | ovalbumin (a model protein) |
| PAS - | periodic acid-Schiff (staining) |
| PBS - | phosphate-buffered saline (buffer) |
| $PLA_2$ - | phospholipase $A_2$ |
| rpm - | rotations-per-minute |
| SPF - | specific pathogen free (conditions) |
| TPA - | 12-O-tetradecanoylphorbol-13-acetate (major ingredient of croton oil) |
| Wt. - | weight |
| w/w - | weight ratio |

## Claims

1. A pharmaceutical composition comprising 7,9-dihydroxy-3-(4,5,7-trihydroxy-2-oxo-2*H*-chromene-3-yl)-4*H*-furo[3,2-*c*]chromen-4-one (**1**):

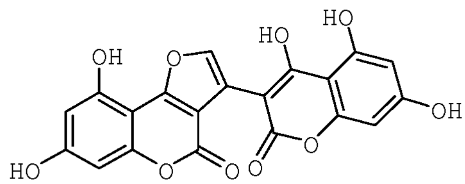

**1**

or a pharmaceutically acceptable salt or hydrate thereof; for use in the therapy of asthma or other inflammatory respiratory diseases.

2. A pharmaceutical composition for use as in claim 1, wherein the inflammatory respiratory diseases are: allergic rhinitis and sinusitis, acute respiratory disease syndrome, and chronic obstructive pulmonary disease.

3. A pharmaceutical composition for use in the therapy of asthma and other inflammatory respiratory diseases according to the previous claims, which consists of:

(1) 7,9-dihydroxy-3-(4,5,7-trihydroxy-2-oxo-2*H*-chromene-3-yl)-4*H*-furo[3,2-*c*]chromen-4-one (**1**) or a pharmaceutically acceptable salt or hydrate thereof as an active pharmaceutical ingredient;

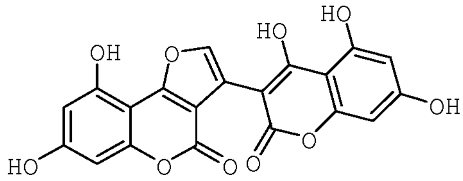

**1**

and
(2) one or more pharmaceutical excipients, required to yield final dosage forms suitable for therapeutic administration that are selected from the group comprising: fillers, diluents, emollients, emulsifiers, binders, disintegrants, lubricants, humectants, thickeners, chelating agents, preservatives and antioxidants.

## Patentansprüche

1. Pharmazeutische Zusammensetzung, umfassend 7,9-Dihydroxy-3-(4,5,7-Trihydroxy-2-Oxo-2H-Chromen-3-yl) -4H-Furo[3,2-c] Chromen-4-eins (1):

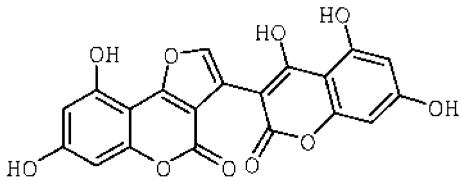

**1**

oder ein pharmazeutisch annehmbares Salz oder Hydrat davon; zur Verwendung in der Therapie von Asthma oder anderen entzündlichen Atemwegserkrankungen.

2. Pharmazeutische Zusammensetzung zur Verwendung nach Anspruch 1, wobei die entzündlichen Atemwegserkrankungen Folgende sind: allergische Rhinitis und Sinusitis, akute Atemwegssyndrom und chronisch obstruktive Lungenerkrankung.

3. Pharmazeutische Zusammensetzung zur Verwendung in der Therapie von Asthma und andere entzündlichen Atemwegserkrankungen nach einem der vorstehenden Ansprüche, die aus Folgendem besteht:

(1) 7,9-Dihydroxy-3-(4,5,7-Trihydroxy-2-Oxo-2H-Chromen-3-yl)-4H-Furo[3,2-c] Chromen-4-eins (1) oder einem pharmazeutisch annehmbaren Salz oder Hydrat davon als pharmazeutischer Wirkstoff;

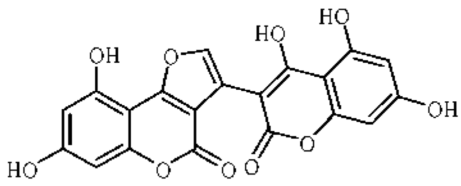

1

und
(2) einem oder mehreren pharmazeutischen Trägerstoffen, die notwendig sind, um finale für therapeutische Verabreichung geeignete Dosierungsformen hervorzubringen, die ausgewählt sind aus der Gruppe, umfassend: Füllstoffe, Verdünnungsmittel, Emollienzien, Emulgatoren, Bindemitteln, Zerfallsmittel, Schmiermittel, Fechthaltemittel, Verdickungsmittel, chelatbildende Mittel, Konservierungsmittel und Antioxidanzien.

## Revendications

1. Composition pharmaceutique comprenant de la 7,9-dihydroxy-3-(4,5,7-trihydroxy-2-oxo-2H-chromène-3-yl)-4H-furo[3,2-c] chromèn-4-one (1) :

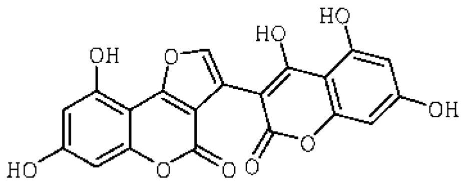

1

ou un sel ou hydrate pharmaceutiquement acceptable de celle-ci ; pour utilisation dans la thérapie de l'asthme ou d'autres maladies respiratoires inflammatoires.

2. Composition pharmaceutique pour utilisation selon la revendication 1, dans laquelle les maladies respiratoires inflammatoires sont : la rhinite et la sinusite allergiques, le syndrome de la maladie respiratoire aiguë, et la bronchopneumopathie chronique obstructive.

3. Composition pharmaceutique pour utilisation dans la thérapie de l'asthme ou d'autres maladies respiratoires inflammatoires selon les revendications précédentes, qui se compose de :

(1) la 7,9-dihydroxy-3-(4,5,7-trihydroxy-2-oxo-2H-chromène-3-yl)-4H-furo[3,2-c]chromèn-4-one (1) ou un sel ou hydrate pharmaceutiquement acceptable de celle-ci en tant que principe pharmaceutiquement actif ;

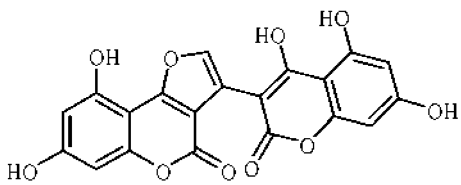

1

et
(2) un ou plusieurs excipients pharmaceutiques, requis pour produire des formes posologiques finales appropriées pour l'administration thérapeutique qui sont sélectionnés parmi le groupe comprenant : des charges, des diluants, des émollients, des émulsifiants, des liants, des délitants, des lubrifiants, des humectants, des épaississants, des agents chélateurs, des conservateurs et des antioxydants.

**Anti-inflammatory activity of 7,9-dihydroxy-3-(4,5,7-trihydroxy-2-oxo-2*H*-chromene-3-yl)-4*H*-furo[3,2-*c*]chromen-4-one (1) in the experimental *in vivo* model of croton oil-induced ear edema in mice**

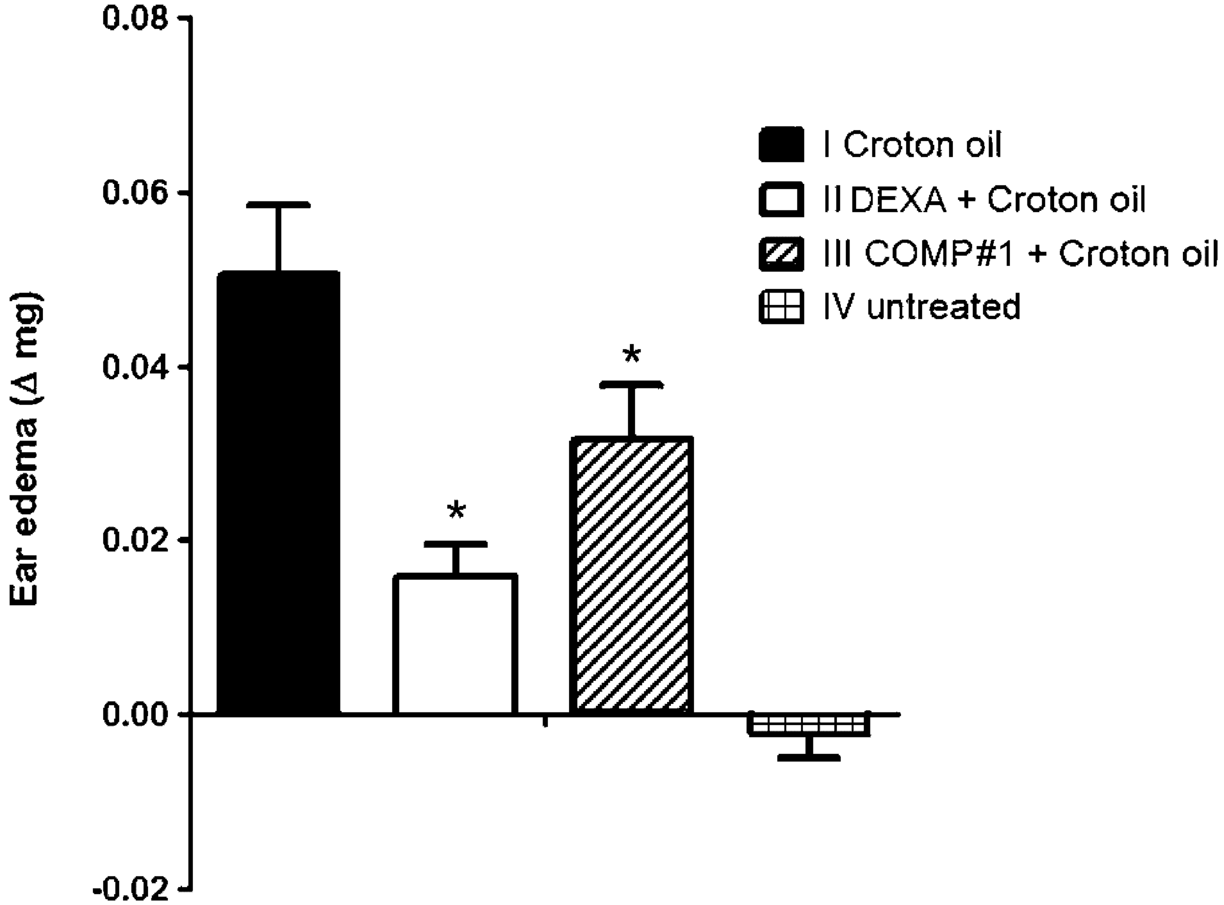


**Figure 1**

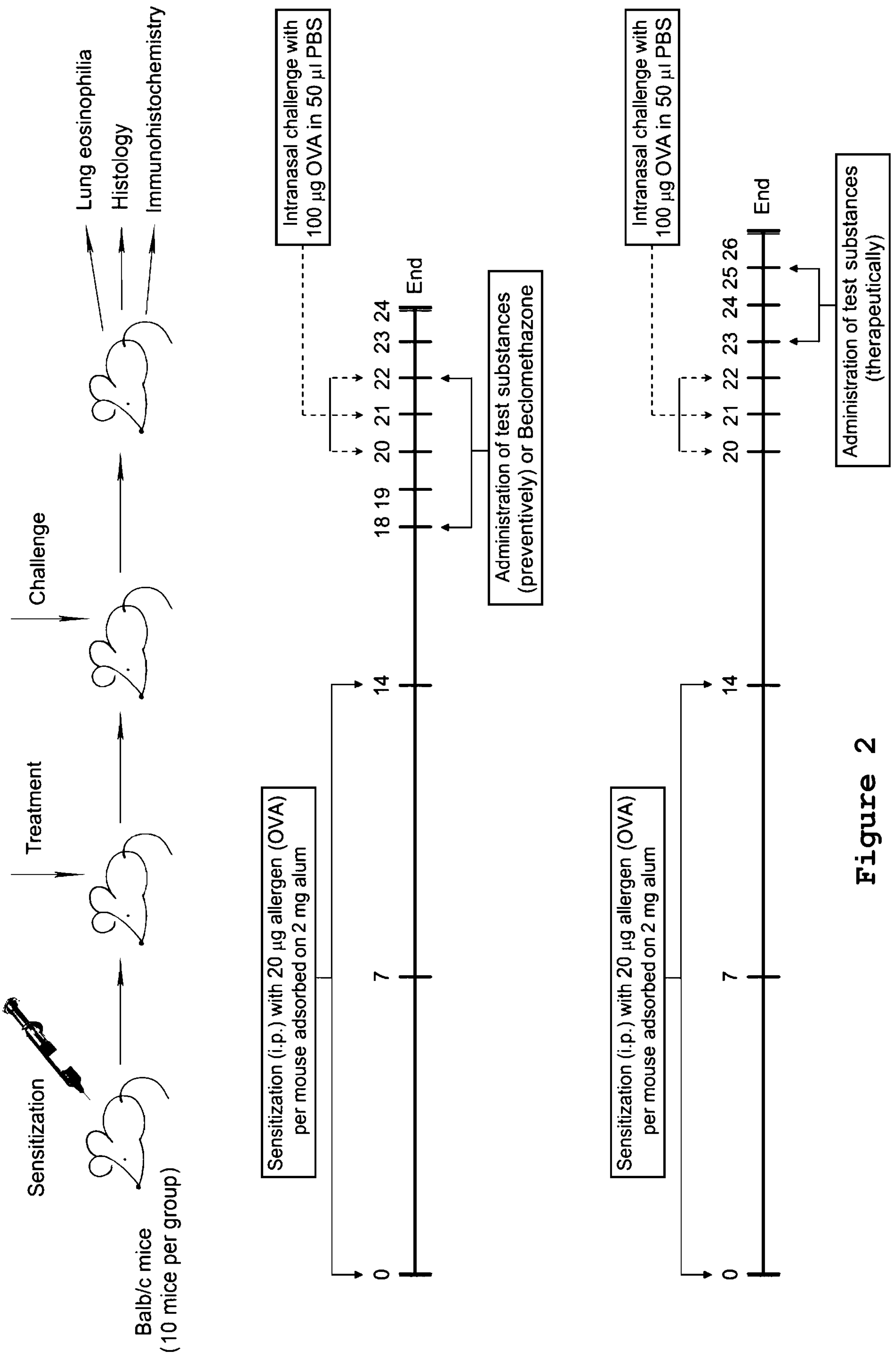
Sensitization
Treatment
Challenge
Lung eosinophilia
Histology
Immunohistochemistry
Balb/c mice
(10 mice per group)
Sensitization (i.p.) with 20 µg allergen (OVA) per mouse adsorbed on 2 mg alum
Intranasal challenge with 100 µg OVA in 50 µl PBS
0
7
14
18 19 20 21 22 23 24
End
Administration of test substances (preventively) or Beclomethazone
Sensitization (i.p.) with 20 µg allergen (OVA) per mouse adsorbed on 2 mg alum
Intranasal challenge with 100 µg OVA in 50 µl PBS
0
7
14
20 21 22 23 24 25 26
End
Administration of test substances (therapeutically)

Figure 2

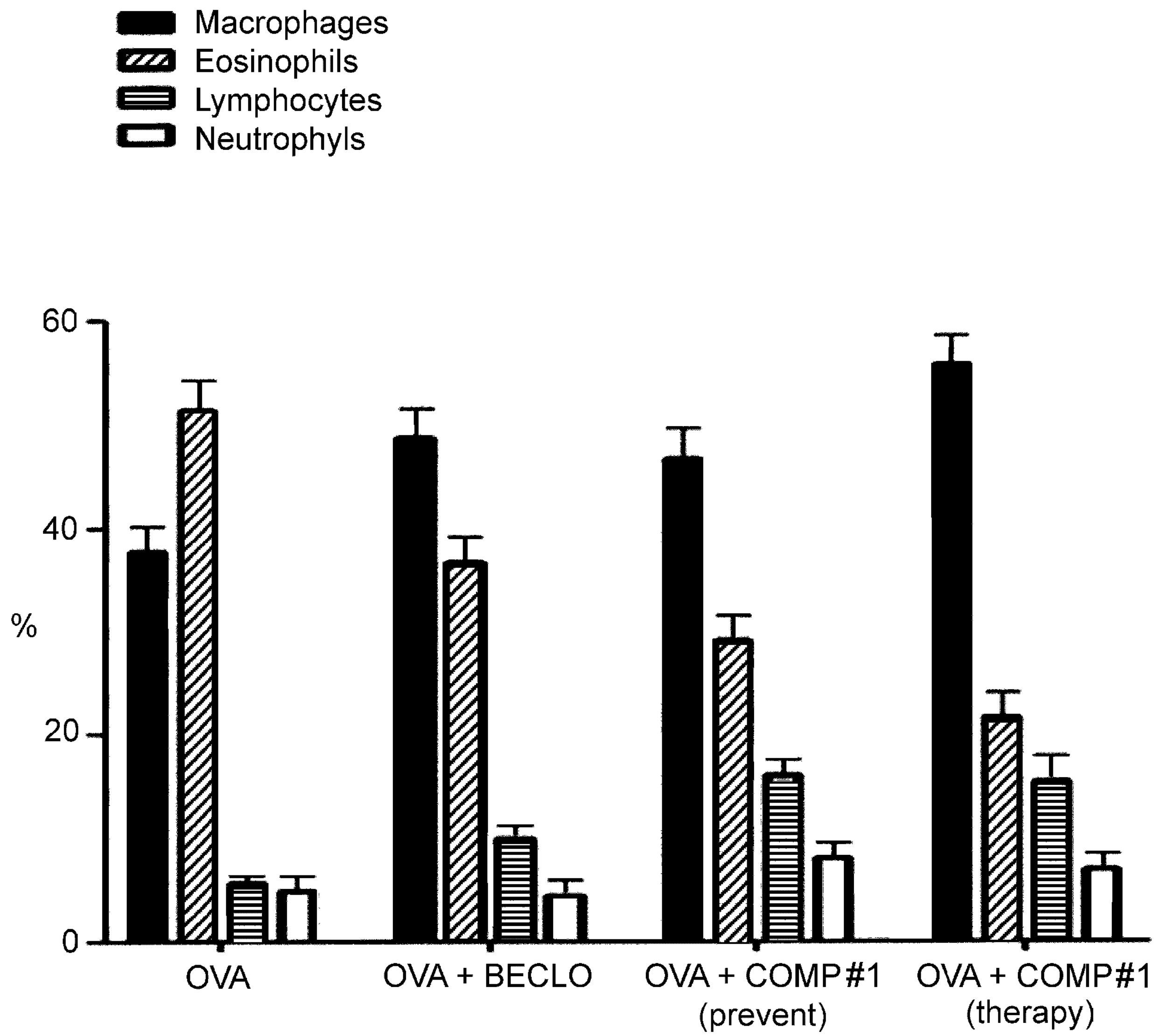
Macrophages
Eosinophils
Lymphocytes
Neutrophyls
60
40
20
0
%
OVA
OVA + BECLO
OVA + COMP#1
(prevent)
OVA + COMP#1
(therapy)

Figure 3A

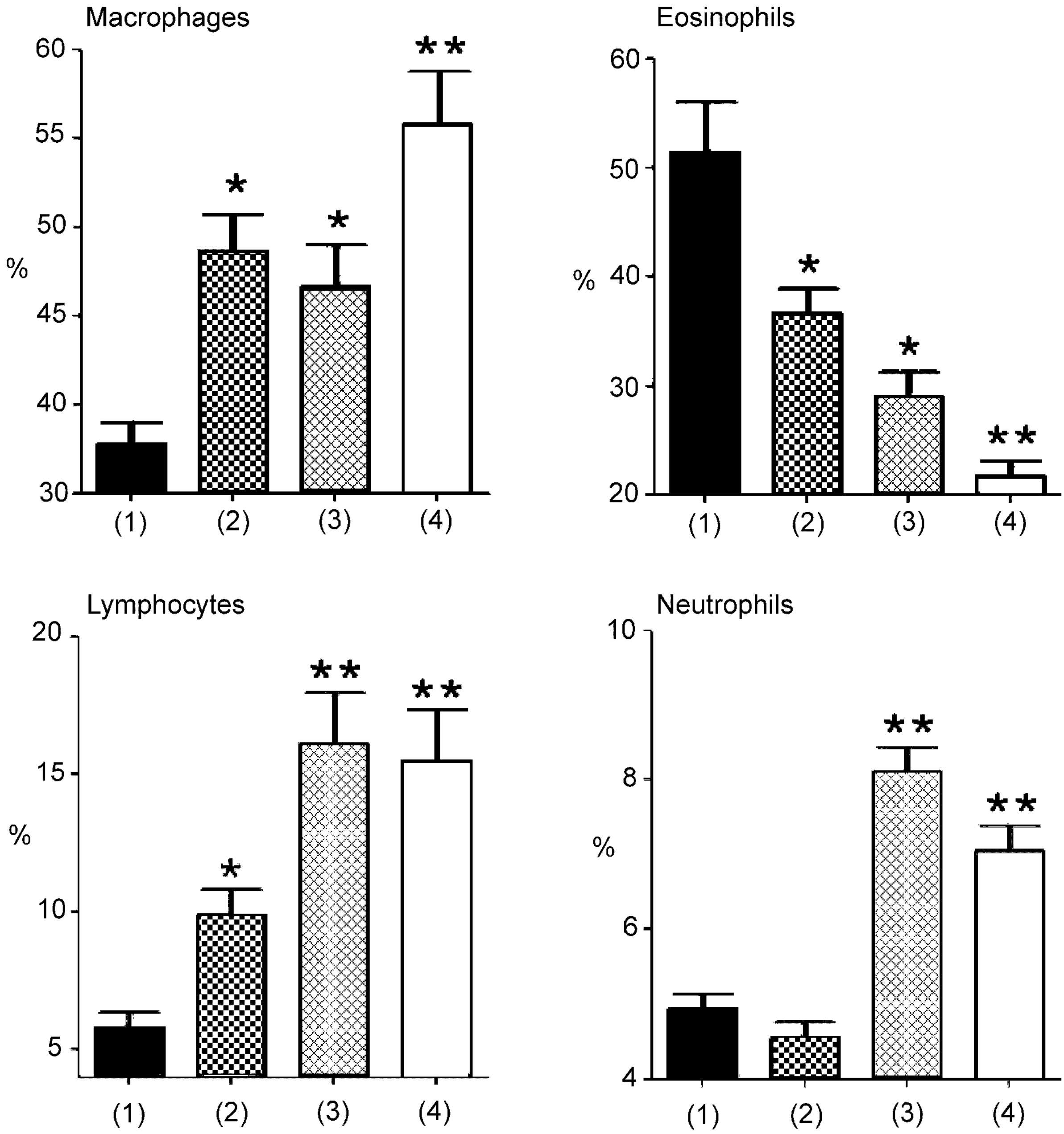


(1) ... OVA
(2) ... OVA + BECLO
(3) ... OVA + COMP#1 (prevent)
(4) ... OVA + COMP#1 (therapy)

Figure 3B

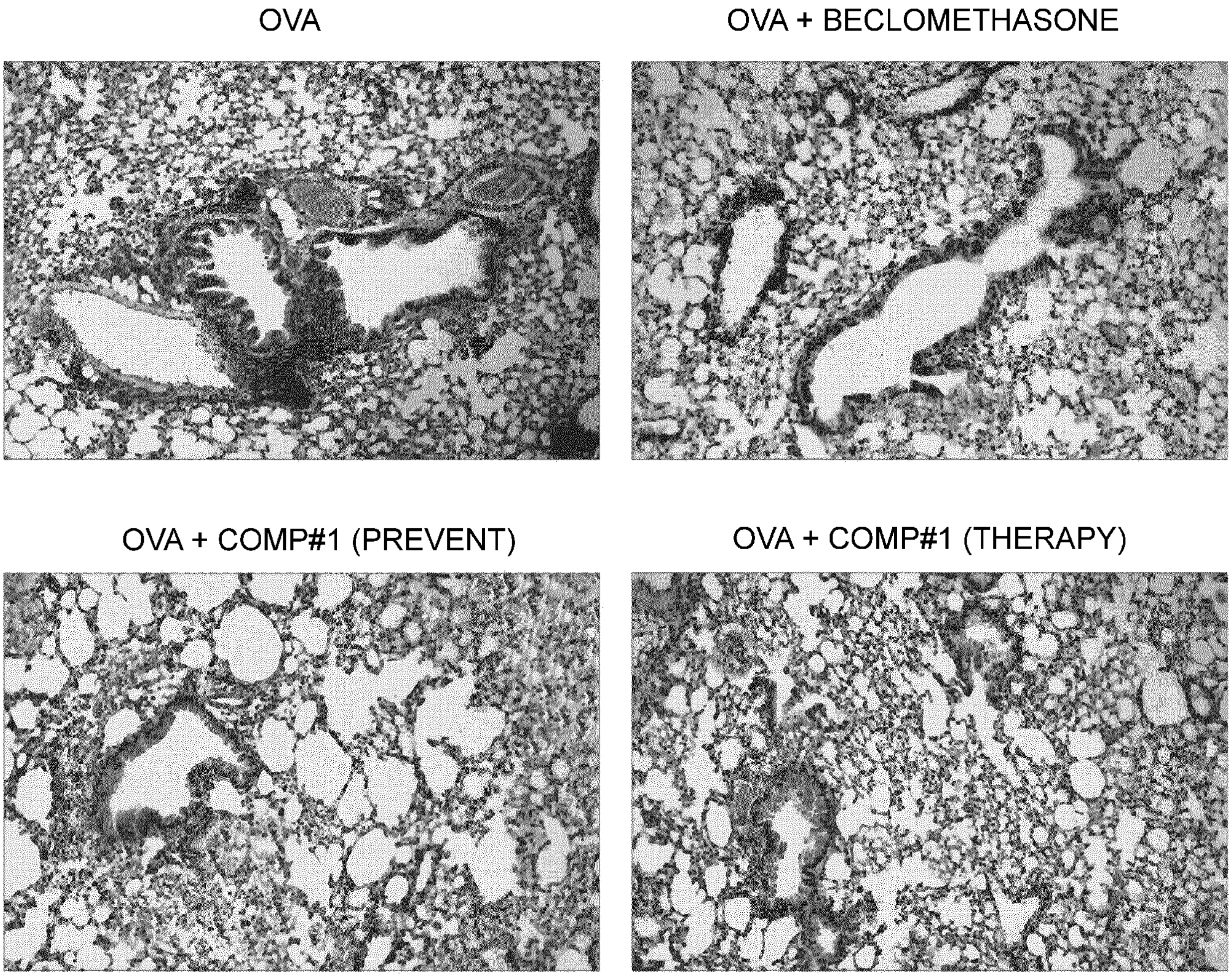
OVA
OVA + BECLOMETHASONE
OVA + COMP#1 (PREVENT)
OVA + COMP#1 (THERAPY)

**Figure 4**

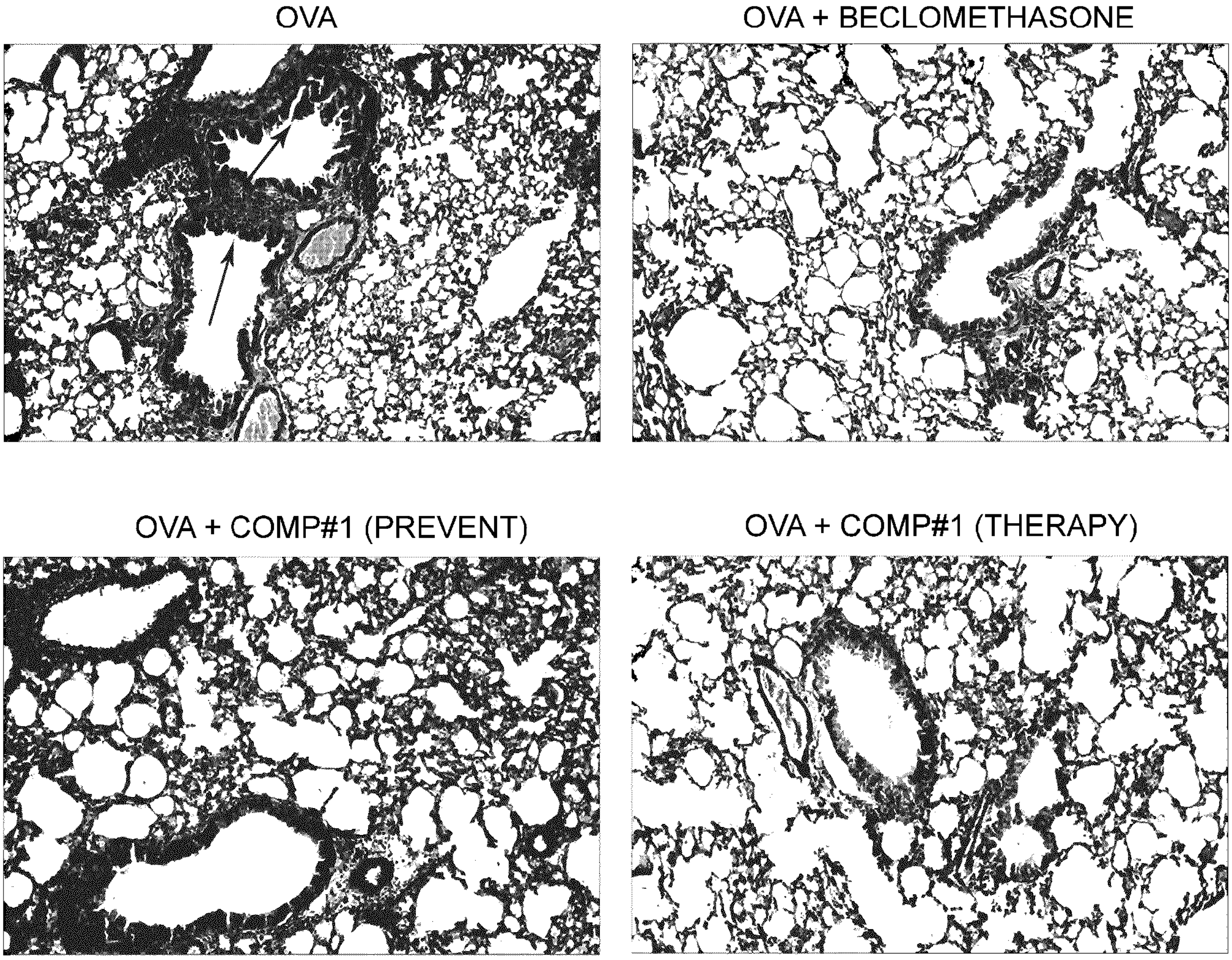
OVA
OVA + BECLOMETHASONE
OVA + COMP#1 (PREVENT)
OVA + COMP#1 (THERAPY)

Figure 5

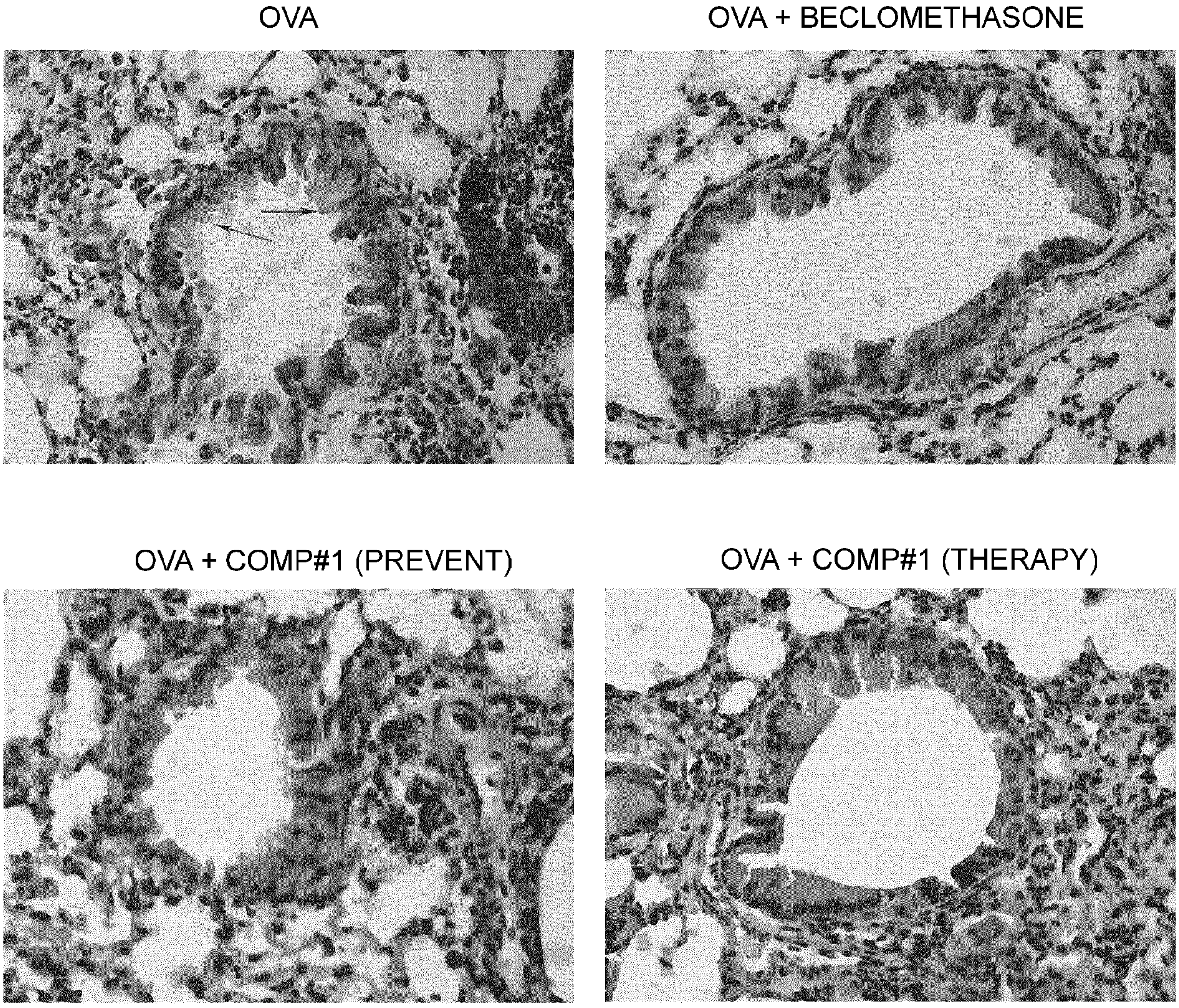
OVA
OVA + BECLOMETHASONE
OVA + COMP#1 (PREVENT)
OVA + COMP#1 (THERAPY)

Figure 6

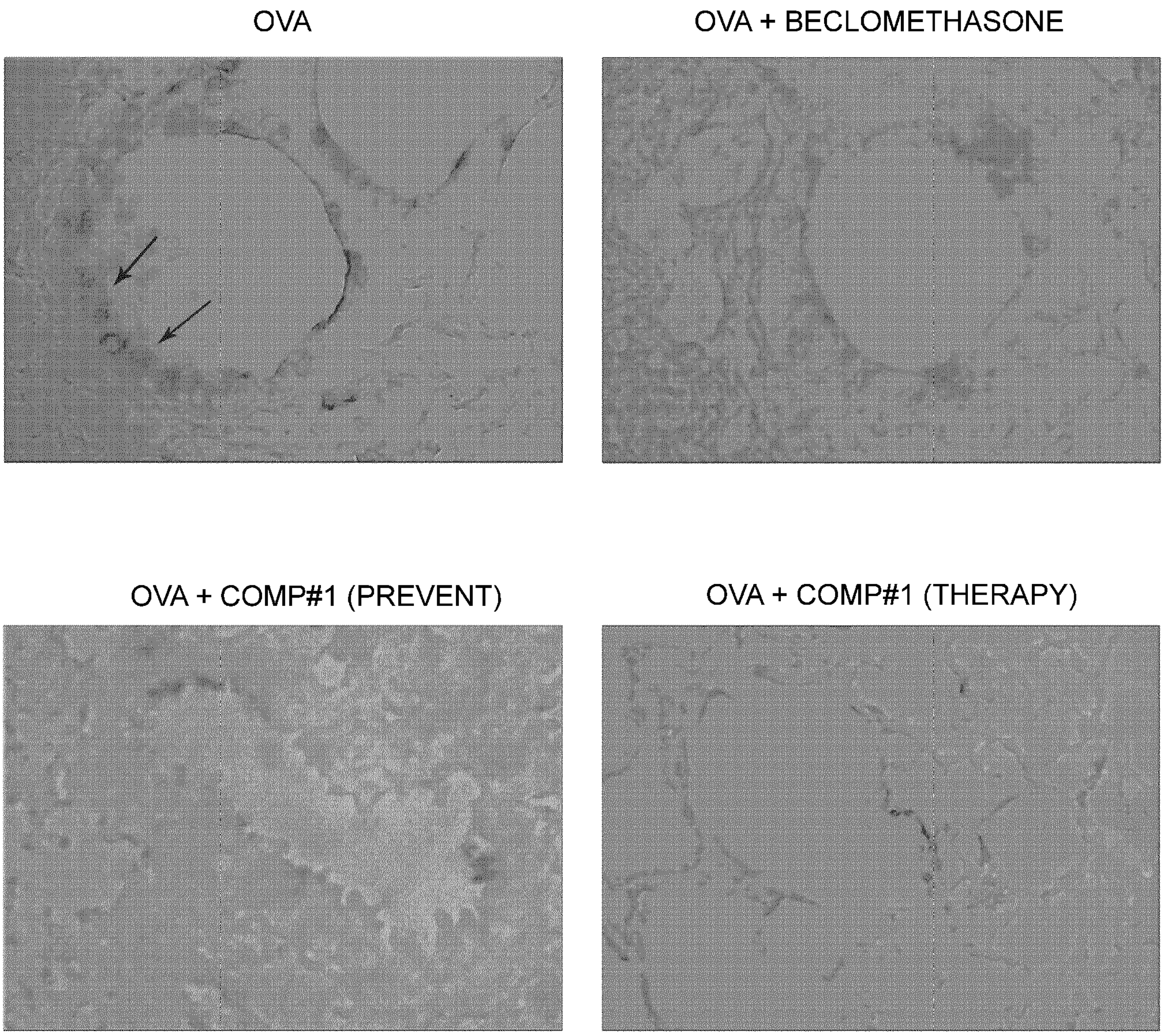
OVA
OVA + BECLOMETHASONE
OVA + COMP#1 (PREVENT)
OVA + COMP#1 (THERAPY)

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005010007 A1
- WO 2005095411 A1
- WO 2016156888 A1


### Non-patent literature cited in the description


- **M. SALTER ; K. BIGGADIKE ; J. L. MATTHEWS ; M. R. WEST ; M. V. HAASE ; S. N. FARROW ; I. J. UINGS ; D. W. GRAY.** Pharmacological properties of the enhanced-affinity glucocorticoid fluticasone furoate in vitro and in vivo model of respiratory inflammatory disease. *Am. J. Physiol. Lung Cell Mol. Physiol.,* 2007, vol. 293, L660-L667
- **K. F. KERREBIJN.** Use of topical corticosteroids in the treatment of childhood asthma. *Am. Rev. Respir. Dis.,* 1990, vol. 141, S77-S81
- **F. BORGES ; F. ROLEIRA ; N. MILHAZES ; L. SANTANA ; E. URIARTE.** Simple Coumarins and Analogues in Medicinal Chemistry: Occurence, Synthesis and Biological Activity. *Curr. Med. Chem.,* 2005, vol. 12, 887-916
- Synthesis of novel hydroxycoumarin derivatives as possible HIV-1 protease inhibitors (in Croatian). **Z. IVEZIC.** Ph.D. Thesis. Pliva Inc, 2000
- **J. SWARBRICK ; J. C. BOYLAN.** Encyclopedia of pharmaceutical technology. M. Dekker, 1998
- **P. O'BRIEN ; J. HASKINGS.** In vitro cytotoxicity assessment. *Methods Mol. Biol.,* 2006, vol. 356, 415-425
- *J. Immunol. Methods,* 1983, vol. 65, 55-63
- **E. PROKSCH ; J. BRANDNER ; J. JENSEN.** The skin: an indispensable barrier. *Experimental Dermatology,* 2008, vol. 12, 1063-1072
- **C. DE BENEDICTIS ; S. JOUBEH ; G. ZHANG ; M. BARRIA ; R. GHOHESTANI.** Immune functions of the skin. *Clinics in Dermatology,* 2001, vol. 5, 573-585
- **L. DE YOUNG ; J. KHEIFETS ; S. BALLARON ; J. YOUNG.** Edema and cell infiltration in the phorbol ester-treated mouse ear are temporally separate and can be differentially modulated by pharmacologic agents. *Agents Actions,* 1989, vol. 26, 335-341
- **A. TUBARO ; P. DRI ; G. DELBELLO ; C. ZILLI ; R. DELLA LOGGIA.** The croton oil ear test revisited. *Agents Actions,* 1986, vol. 17, 347-349
- **G. CORUZZI ; C. POZZOLI ; M. ADAMI ; D. GRANDI ; N. GUIDO ; R. SMITS ; I. DE ESCH ; R. LEURS.** Strain-dependent effects of the histamine H4 receptor antagonist JNJ7777120 in a murine model of acute skin inflammation. *Experimental Dermatology,* 2011, vol. 21, 32-37
- **M. OTUKI ; F. VIEIRA-LIMA ; Â. MALHEIROS ; R. YUNES ; J. CALIXTO.** Topical antiinflammatory effects of the ether extract from Protium kleinii and $\alpha$-amyrin pentacyclic triterpene. *European Journal of Pharmacology,* 2005, vol. 507, 253-259
- **X. WANG ; M. LAN ; H. WU ; Y. SHI ; J. LU ; J. DING ; K. WU ; J. JIN ; D. FAN.** Direct effect of croton oil on intestinal epithelial cells and colonic smooth muscle cells. *World Journal of Gastroenterology,* 2002, vol. 8, 103-107
- **M. DENNING.** Epidermal keratinocytes: regulation of multiple cell phenotypes by multiple protein kinase C isoforms. *International Journal of Biochemistry and Cell Biology,* 2004, vol. 36, 1141-1146
- **K. HON ; V. LEE ; T. LEUNG ; K. LEE ; A. CHAN ; T. FOK ; P. LEUNG.** Corticosteroids are not present in a traditional Chinese medicine formulation for atopic dermatitis in children. *Annals of the Academy of Medicine Singapore,* 2006, vol. 35, 759-763
- **N. HANANIA.** Targeting airway inflammation in asthma - current and future therapies. *Chest,* 2008, vol. 133, 989-998
- **R. DJUKANOVIC ; W. ROCHE ; J. WILSON ; C. BEASLEY ; O. TWENTYMAN ; P. HOWARTH ; H. HOLGATE.** Mucosal inflammation in asthma. *Am. Rev. Respir Dis.,* 1990, vol. 142, 434-457
- **T. MATSUMOTO ; Y. ASHIDA ; R. TSUKUDA.** Pharmacological modulation of immediate and late airways response and leukocyte infiltration in the guinea pig. *J. Pharm. Exp. Ther.,* 1994, vol. 269, 1236-1244

- **M. AZZAWI ; B. BRADLEY ; P. JEFFERY ; A. FREW ; A. WARDLAW ; G. KNOWLWA ; B. ASSOUFI ; J. COLLINS ; S. DURHAM ; A. KAY.** Identification of activated lymphocytes and eosinophils in bronchial biopsies in stable atopic asthma. *Am. Rev. Respir. Dis.,* 1990, vol. 142, 1407-1418
- **D. DALTON ; K. PITTS-MEE.** Multiple defects of immune cell function in mice with disputed interferon-g genes. *Science (Wash DC),* 1993, vol. 259, 1739-1742
- **K. ARAI ; F. LEE ; A. MIYAJIMA.** Cytokines: Coordinators of immune and inflammatory response. *Annu. Rev. Biochem.,* 1990, vol. 59, 783-836
- **C. CORRIGAN ; A. KAY.** T cells and eosinophils in the pathogenesis of asthma. *Immunol. Today,* 1992, vol. 13, 501-507
- **F. DAUBEUF ; N. FROSSARD.** Performing Bronchoalveolar Lavage in the Mouse. *Curr. Protoc. Mouse Biol.,* 2012, vol. 2, 167-175